**Europäisches Patentamt**

**European Patent Office**

**Office européen des brevets**

(19)

(11) **EP 0 726 729 B1**

(12) **EUROPÄISCHE PATENTSCHRIFT**

(45) Veröffentlichungstag und Bekanntmachung des Hinweises auf die Patenterteilung:
16.04.1997 Patentblatt 1997/16

(21) Anmeldenummer: 94931505.5

(22) Anmeldetag: 29.10.1994

(51) Int. Cl.$^6$: **A61B 5/00**, G01N 21/47

(86) Internationale Anmeldenummer:
PCT/DE94/01290

(87) Internationale Veröffentlichungsnummer:
WO 95/12348 (11.05.1995 Gazette 1995/20)

(54) **VERFAHREN UND VORRICHTUNG ZUR ANALYSE VON GLUCOSE IN EINER BIOLOGISCHEN MATRIX**

PROCESS AND DEVICE FOR DETERMINING THE GLUCOSE CONCENTRATION IN A BIOLOGICAL MATRIX

PROCEDE ET DISPOSITIF PERMETTANT DE DETERMINER LA CONCENTRATION DE GLUCOSE DANS UNE MATRICE BIOLOGIQUE

(84) Benannte Vertragsstaaten:
AT BE CH DE DK ES FR GB GR IE IT LI LU NL PT SE

(30) Priorität: 04.11.1993 DE 4337570

(43) Veröffentlichungstag der Anmeldung:
21.08.1996 Patentblatt 1996/34

(73) Patentinhaber: BOEHRINGER MANNHEIM GMBH
68298 Mannheim (DE)

(72) Erfinder:
• ESSENPREIS, Matthias
D-82131 Gauting (DE)
• BÖCKER, Dirk
D-69115 Heidelberg (DE)
• HEIN, Heinz-Michael
D-64342 Seeheim-Jugenheim (DE)
• HAAR, Hans-Peter
D-69168 Wiesloch (DE)

(74) Vertreter: Pfeifer, Hans-Peter, Dr.,
Dr. H.-P. Pfeifer Dr. P. Jany,
Patentanwälte et al
Beiertheimer Allee 19
76137 Karlsruhe (DE)

(56) Entgegenhaltungen:
EP-A- 0 280 986          US-A- 4 972 331
US-A- 5 119 815          US-A- 5 119 819

**Beschreibung**

Die Erfindung betrifft ein Verfahren und eine Vorrichtung zur Analyse von Glucose in einer biologischen Matrix.

Der Begriff "biologische Matrix" bezeichnet eine Körperflüssigkeit oder ein Gewebe eines lebenden Organismus. Biologische Matrices, auf die sich die Erfindung bezieht, sind optisch heterogen, d.h. sie enthalten eine Vielzahl von Streuzentren, an denen eingestrahltes Licht gestreut wird. Im Falle von biologischem Gewebe, insbesondere Hautgewebe, werden die Streuzentren von den Zellwänden und anderen in dem Gewebe enthaltenen optisch heterogenen Bestandteilen gebildet.

Körperflüssigkeiten, insbesondere Blut, sind ebenfalls optisch heterogene biologische Matrices, weil sie Partikel enthalten, an denen die Primärstrahlung vielfach gestreut wird. Auch Milch und andere in der Lebensmittelchemie zu untersuchende Flüssigkeiten enthalten vielfach eine hohe Konzentration von Streuzentren, beispielsweise in Form von emulgierten Fetttröpfchen.

Zur qualitativen und quantitativen analytischen Bestimmung von Komponenten solcher biologischen Matrices werden im allgemeinen Reagenzien bzw. Reagenziensysteme eingesetzt, deren Reaktion mit der jeweiligen Komponente zu einer physikalisch nachweisbaren Änderung der Reaktionslösung, beispielsweise einer Änderung ihrer Farbe führt, die als Meßgröße gemessen werden kann. Durch Kalibration mit Standardproben bekannter Konzentration wird eine Korrelation zwischen den bei unterschiedlichen Konzentrationen gemessenen Werten der Meßgröße und der jeweiligen Konzentration bestimmt.

Diese Verfahren ermöglichen zwar Analysen mit hoher Genauigkeit und Empfindlichkeit, machen es jedoch erforderlich, eine flüssige Probe, insbesondere eine Blutprobe, zur Analyse dem Körper zu entnehmen ("Invasive Analyse"). Diese Probenentnahme ist unangenehm und schmerzhaft und führt zu einem gewissen Infektionsrisiko.

Dies gilt vor allem, wenn eine Krankheit sehr häufige Analysen erforderlich macht. Das wichtigste Beispiel ist der Diabetes mellitus. Um schwere Folgeerkrankungen und kritische Zustände des Patienten zu vermeiden, ist es bei dieser Krankheit erforderlich, den Glucosegehalt des Blutes sehr häufig oder sogar kontinuierlich zu bestimmen.

Es sind deshalb bereits eine Vielzahl von Verfahren und Vorrichtungen vorgeschlagen worden, um Glucose in Blut, Gewebe oder anderen biologischen Matrices in vivo und nicht-invasiv zu bestimmen.

Ein Überblick über physikochemische (reagenzienfreie) Bestimmungen von Glucose in vivo wird gegeben in: J.D. Kruse-Jarres "Physicochemical Determinations of Glucose in vivo", J. Clin. Chem. Clin. Biochem. 26 (1988), 201-208. Als nicht-invasive Verfahren werden dabei unter anderem die Kernspinresonanz (NMR, nuclear magnetic resonance), Elektronenspinresonanz (ESR, electron spin resonance) sowie die Infrarotspektroskopie genannt. Keines dieser Verfahren hat jedoch bis jetzt praktische Bedeutung erlangen können. Teilweise sind extrem große und aufwendige Apparaturen erforderlich, die für die Routineanalytik oder gar die Selbstkontrolle des Patienten (home monitoring) völlig ungeeignet sind.

Die Erfindung bezieht sich auf eine Teilgruppe solcher Verfahren, bei denen Licht von einem Lichtsender durch eine die biologische Matrix begrenzende Grenzfläche als Primärlicht in diese eingestrahlt und aus der biologischen Matrix durch eine diese begrenzende Grenzfläche austretendes Licht von einem Lichtempfänger detektiert wird, um eine durch die Wechselwirkung mit der biologischen Matrix (ohne Verwendung von Reagenzien) veränderliche physikalische Eigenschaft des Lichts zu bestimmen, die mit der Konzentration von Glucose in der biologischen Matrix korreliert. Ein solcher Verfahrensschritt wird nachfolgend als "Detektionsschritt" bezeichnet.

Die in einem Detektionsschritt bestimmte (detektierte) mit der Glucosekonzentration korrelierende physikalische Eigenschaft des Lichtes, die man auch als "quantifizierbarer Parameter" (englisch: quantifiable parameter) bezeichnen kann, wird nachfolgend einfachheitshalber als "Meßgröße" bezeichnet. Dieser Begriff darf aber nicht dahingehend verstanden werden, daß ein bestimmter Betrag der Meßgröße in einer entsprechenden Maßeinheit gemessen werden muß.

Da die hier diskutierten Verfahren keine Absolutmessung der Glucosekonzentration ermöglichen, ist stets (ebenso wie bei den gebräuchlichen, auf chemischen Reaktionen basierenden Analyseverfahren) eine Kalibration erforderlich. Üblicherweise wird in mindestens einem Kalibrationsschritt, der meßtechnisch in gleicher Weise wie der Detektionsschritt ausgeführt wird, die Glucosekonzentration an einer biologischen Matrix mit bekannter Glucosekonzentration bestimmt. Dabei kann die jeweilige Glucosekonzentration in der biologischen Matrix mit irgendeinem vorbekannten Verfahren zur Bestimmung der absoluten Konzentration der Glucose ermittelt werden.

In einem Auswerteschritt des Analyseverfahrens wird die Glucosekonzentration aus der Änderung der Meßgröße bei mindestens einem Detektionsschritt im Vergleich zu mindestens einem Kalibrationsschritt ermittelt. Der Auswerteschritt umfaßt einen Auswertealgorithmus, in dem die Glucosekonzentration in vorbestimmter Weise aus den Ergebnissen von mindestens einem Detektionsschritt und mindestens einem Kalibrationsschritt ermittelt wird.

Die Wellenlängen des Lichts, die für solche Verfahren diskutiert werden, liegen allgemein zwischen etwa 300 nm und mehreren tausend nm, also im Spektralbereich zwischen dem nahen UV- und infrarotem Licht. Der Begriff "Licht" darf nicht als Einschränkung auf den sichtbaren Spektralbereich des Lichtes verstanden werden.

Nahezu alle bekannten Verfahren dieser Art basieren auf den Prinzipien der Spektroskopie. Grundlage ist dabei die Wechselwirkung des eingestrahlten Primärlichtes mit Vibrations- und Rotationszuständen der zu analysierenden Moleküle. Die Meßgröße ist dabei die Lichtintensität I, deren Abnahme durch Absorption in der biologischen Matrix in Abhängigkeit von der Wellenlänge L bestimmt wird. Üblicherweise wird die Schwächung des Lichts als Extinktion $E(L) = lg [I(L)/I_0(L)]$ ausgedrückt, wobei I die Intensität des Sekundärlichtes und $I_0$ die Intensität des Primärlichtes bezeichnen. Um die Änderungen des Spektrums mit hinreichender Genauigkeit detektieren zu können, muß in dem Detektionsschritt ein hinreichender breiter Spektralbereich mit guter Wellenlängen-Auflösung erfaßt werden. Im allgemeinen wird die Extinktion bei mindestens zwei Wellenlängen, die sich um mindestens etwa 50 nm unterscheiden, mit einer Bandbreite des Lichts von weniger als 5 nm gemessen.

Die Vibrations- und Rotations-Grundzustände der Glucose befinden sich im IR-Bereich bei Wellenlängen von mehr als 2500 nm. Sie können wegen der starken Absorption des in biologischen Matrices stets in hoher Konzentration gegenwärtigen Wassers nicht für die nicht-invasive Analyse von Glucose verwendet werden. Im Bereich des nahen Infrarot (NIR) ist die Absorption des Wassers geringer (sogenanntes "Wasser-Transmissionsfenster"). Die spektrale Analyse von Glucose in diesem Bereich basiert auf der Absorption durch Obertöne (overtones) und Kombinationsschwingungen der Vibrations- und Rotationsgrundzustände des Glucosemoleküls (vgl. vorstehend zitierter Artikel von Kruse-Jarres und EP-A-0 426 358).

Die praktische Realisierung eines nicht-invasiven Glucose-Sensors auf Basis dieser Prinzipien verursacht außerordentlich große Schwierigkeiten, die vor allem daraus resultieren, daß das Nutzsignal (die Änderung des Absorptions-Spektrums in Abhängigkeit von einer Änderung der Glucosekonzentration) sehr gering ist und diesem kleinen Nutzsignal ein großer Hintergrund von Störsignalen gegenübersteht, die insbesondere von der spektralen Absorption von Wasser und anderen stark absorbierenden Komponenten (unter anderem dem roten Blutfarbstoff Hämoglobin) resultieren.

Zur Lösung dieses Problems wurden zahlreiche unterschiedliche Versuche unternommen. Vielfach sollen dabei die Störeinflüsse durch eine geeignete Wahl der Meßwellenlänge in Verbindung mit einer Differenzmessung eliminiert werden. Weit verbreitet ist vor allem die "Zwei-Wellenlängen-Spektroskopie", bei der eine erste Meßwellenlänge so gewählt ist, daß die Glucose dort möglichst stark absorbiert, während eine zweite Wellenlänge als Referenzwellenlänge so gewählt ist, daß die Lichtabsorption möglichst weitgehend unabhängig von der Glucosekonzentration ist. Solche oder ähnliche Verfahren sind beispielsweise Gegenstand der EP-A-0 160 768, der WO 93/00856 und des US-Patentes 5,028,787.

Trotz dieser Bemühungen ist es bisher nicht gelungen, einen praktisch funktionsfähigen nicht-invasiven Glucosesensor zur Verfügung zu stellen.

Wesentlich realistischer ist die Möglichkeit, Substanzen, die optisch um mehrere Größenordnungen stärker als Glucose absorbieren, mit einem auf den Prinzipien der Spektralanalyse basierenden In-vivo-Sensor zu analysieren. Das wichtigste Beispiel ist die Bestimmung des Hämoglobins (Hb) bzw. dessen oxidierter Form $HbO_2$. Da diese Parameter Auskunft über den Oxigenierungszustand des Blutes geben, werden solche Sensoren auch als Oximeter bezeichnet. Aus der Literatur sind zahlreiche unterschiedliche Konstruktionen und Verfahren für nicht-invasive Oximeter bekannt, wobei überwiegend mit der Zwei-Wellenlängen-Spektroskopie gearbeitet wird. Verwiesen sei beispielsweise auf WO 89/01758, EP-A-0 286 142, EP-A-0 353 619, WO 91/17697 und das US-Patent 5,057,695.

In der europäischen Patentschrift 0 074 428 ist ein Verfahren und eine Vorrichtung zur quantitativen Bestimmung von Glucose durch Laser-Lichtstreuung beschrieben. Dabei wird davon ausgegangen, daß die Glucosemoleküle einen durch die Lösung transmittierten Lichtstrahl streuen und daß sich daraus die Glucosekonzentration ableiten läßt. Entsprechend dieser Theorie wird die Raumwinkelverteilung der aus einer Untersuchungsküvette oder einem untersuchten Körperteil austretenden transmittierten Lichtintensität als mit der Glucosekonzentration korrelierende Meßgröße verwendet. Insbesondere wird die Intensität des transmittierten Lichtes in einem Winkelbereich, in dem die Änderung in Abhängigkeit von der Glucosekonzentration möglichst groß ist, gemessen und in Beziehung zu der an dem Zentralstrahl, welcher die Probe in gerader Richtung durchdringt, gemessenen Intensität gesetzt.

Der Erfindung liegt die Aufgabe zugrunde, ein Verfahren für die analytische Bestimmung von Glucose in einer biologischen Matrix zur Verfügung zu stellen, welches mit einfachen Mitteln, reagenzienfrei und nicht-invasiv arbeitet und eine gute Analysegenauigkeit, zum Beispiel für die Beobachtung der Änderung der Analytkonzentration (Verlaufskontrolle) über einen ausreichenden Zeitraum, ermöglicht.

Die Aufgabe wird bei einem Verfahren, welches mindestens einen Detektionsschritt und einen Auswerteschritt im Sinne der vorstehenden Erläuterungen umfaßt, dadurch gelöst, daß als mit der Glucose-Konzentration korrelierende meßbare Eigenschaft (d.h. als Meßgröße im vorstehend erläuterten Sinn) ein der Laufzeit des Lichtes innerhalb der biologischen Matrix zwischen einem definierten Einstrahlungsort und einem definierten Detektionsort entsprechender Parameter bestimmt wird. Eine entsprechende Vorrichtung ist ebenfalls Bestandteil der erfindungsgemäßen Problemlösung.

Eine wesentliche Grundlage der Erfindung ist die Erkenntnis, daß der mittlere optische Weg von Photonen innerhalb einer optisch heterogenen biologischen Matrix in überraschend starkem Maß von der Glucosekonzentration beeinflußt wird. Im Rahmen der vorliegenden Erfindung wurde festgestellt, daß diese Abhängigkeit groß genug ist, um die Glucosekonzentration ohne Reagenzien in-vivo mit vertretbarem Aufwand zu bestimmen. Dieser erstaunliche Effekt läßt sich nach dem gegenwärtigen Kenntnisstand der Erfinder wie folgt erklären.

Die Änderung der Glucosekonzentration führt zu einer Änderung des Brechungsindex der in der biologischen Matrix enthaltenen Flüssigkeit, in der die Glucose gelöst ist. Aus der Änderung des Brechungsindex wiederum resultiert eine Änderung der Lichtstreuung an den in der Matrix enthaltenen Streuzentren. Da die Änderung des Brechungsindex pro mmol nur etwa 0,002 % beträgt, ist jeder einzelne Streuprozeß nur in sehr geringem Ausmaß von der Glucosekonzentration abhängig. Im Rahmen der Erfindung wurde festgestellt, daß sich dieser extrem kleine Effekt zur Analyse von Glucose praktisch nutzen läßt, wenn man einen Parameter, der für die Laufzeit von vielfach in der biologischen Matrix gestreuten Photonen charakteristisch ist, erfaßt. Mit anderen Worten ist die mittlere optische Weglänge der Photonen (die das Produkt aus der Laufzeit und der Lichtgeschwindigkeit in der Matrix ist) infolge einer Vielzahl von Streuprozessen zwischen Einstrahlungsort und Detektionsort so stark von der Glucosekonzentration abhängig, daß ein der Laufzeit der Photonen entsprechender Parameter (nachfolgend: "Laufzeit-Parameter") als Maß für die Konzentration der Glucose in der biologischen Matrix verwendet werden kann.

Als Laufzeit-Parameter in diesem Sinne ist jeder quantifizierbare Parameter des Lichtes anzusehen, der mit der Laufzeit korreliert und unter den jeweiligen Meßbedingungen überwiegend von der Laufzeit des Lichtes zwischen Einstrahlungsort und Detektionsort abhängt. Das "Bestimmen" eines Laufzeit-Parameters erfordert (ebenso wie bei bisher gebräuchlichen mit der Glucosekonzentration korrelierenden Meßgrößen) keine Absolutmessung. Es genügt, wenn als Ergebnis des Detektionsschrittes ein mit dem Laufzeit-Parameter eindeutig und reproduzierbar korrelierendes elektrisches Signal gewonnen wird, welches in dem Auswerteschritt in Beziehung zu einem auf gleicher Weise an einer biologischen Matrix mit bekannter Glucosekonzentration bestimmten Wert des gleichen Laufzeit-Parameters gesetzt werden kann.

Gemäß einer ersten Ausführungsform der Erfindung wird die Laufzeit direkt bestimmt, indem bei jeder Detektionsmessung ein kurzer Impuls des Primärlichtes in die biologische Matrix eingestrahlt und der resultierende an dem Detektionsort austretende Impuls detektiert wird. Da die Laufzeit zwischen Einstrahlungsort und Detektionsort sehr kurz ist (sie liegt in der Größenordnung von $10^{-9}$ sec) bedingt dies einen erheblichen meßtechnischen Aufwand.

Bevorzugt ist deswegen eine Verfahrensweise, bei der das Primärlicht mit einer Trägerfrequenz moduliert wird, so daß sich in der biologischen Matrix Lichtintensitätswellen ausbreiten, deren Wellenlänge dem Quotient aus der Lichtgeschwindigkeit in der biologischen Matrix und der Modulationsfrequenz entspricht. Dabei wird die Phasenverschiebung des Sekundärlichtes als Laufzeit-Parameter bestimmt. In einem nichtabsorbierenden Medium ist die Laufzeit dt unmittelbar linear von der Phasenverschiebung $d\Phi$ abhängig ($dt = d\Phi/\Omega$, wobei $\Omega$ die Modulationsfrequenz des Primärlichtes ist). Unter den praktischen Bedingungen der vorliegenden Erfindung ist die Dämpfung durch Absorption verhältnismäßig gering, so daß diese Bedingung in guter Näherung gilt.

Um die erforderliche Analysegenauigkeit zu gewährleisten ist eine Meßtechnik erforderlich, die die Bestimmung extrem kleiner Phasenverschiebungen bei hohen Frequenzen mit sehr guter Empfindlichkeit und Reproduzierbarkeit erlaubt. Solche meßtechnischen Verfahren sind bekannt. Sie werden unter anderem auch für analytische Zwecke zur Bestimmung der Lebensdauer von fluoreszierenden Farbstoffen und für die zeitaufgelöste Spektroskopie (time resolved spectroscopy) verwendet.

Die Bestimmung der Lebensdauer von fluoreszierenden Farbstoffen ist bei analytischen Verfahren gebräuchlich, die darauf basieren, den Analyt durch eine spezifische Bindungsreaktion mit einer fluoreszierenden Markierung zu versehen. Bei einem Teil solcher Verfahren wird die Fluoreszenz-Lebensdauer als Meßgröße bestimmt ("Phasenfluorometrie"). Um die sehr kurzen Lebensdauern (in der Größenordnung von weniger als 100 psec.) bestimmen zu können, wurden Radiofrequenz (RF, radio frequency)-modulierte Spektrometer entwickelt, die auf Basis einer Modulation des Lichtes mit einer Frequenz zwischen etwa 100 MHz und mehreren GHz arbeiten. Ein wichtiges Beispiel ist das Heterodyn-Verfahren, bei dem das von dem Meßempfänger (Detektor) empfangene Signal mit einem zweiten periodischen Signal gemischt wird, dessen Frequenz sich von dem ersten Signal um wenige kHz unterscheidet. Das resultierende Differenzsignal kann verhältnismäßig einfach mit einem Lock-in-Verstärker oder sonstigen schmalbandig selektierenden Verstärkungsverfahren aufbereitet und gemessen werden. Eine leistungsfähige Apparatur für solche Messungen ist beschrieben in J.R. Lakowicz et al.: "2-GHz frequency-domain fluorometer", Rev.Sci.Instrum., 57 (1986), 2499-2506. Ein Verfahren zur Analyse von Glucose mit Hilfe der Phasenmodulations-Flourometrie ist aus J.R. Lakowicz und B. Maliwal: "Optical sensing of glucose using phase-modulation fluorimetry", Analytica Chimica Acta, 271, (1993) 155-164 bekannt. Es handelt sich dabei um einen In-vitro-Test, bei dem die Probe in konventioneller Weise invasiv entnommen und mit den erforderlichen Reagenzien, die eine fluoreszierende Markierung enthalten, gemischt werden muß.

Die zeitaufgelöste Spektroskopie als Verfahren zur Invivo-Analyse geht auf B. Chance zurück und wird von dem Autor vor allem als Weiterentwicklung der Zwei-Wellenlängen-Spektroskopie empfohlen (vgl. die US-Patente 4,972,331, 5,119,815, 5,122,974, 5,167,230 und 5,187,672). Die darin gegebenen Beispiele beziehen sich auf stark absorbierende Substanzen, insbesondere Pigmente und die oben erwähnen Oxigenierungsparameter Hb und $HbO_2$.

Es geht dabei um eine Lösung für ein grundlegendes Problem bei der Spektroskopie streuender Medien (wie beispielsweise biologischer Gewebe), nämlich die Unkenntnis über die optische Weglänge. Deren Kenntnis ist erforderlich, um die gemessenen Absorptionsspektren quantifizieren und die Konzentration eines absorbierenden Stoffes berechnen zu können. In einem nichtstreuenden Medium entspricht die optische Weglänge der Küvettenlänge. In einem streuenden Medium ist sie dagegen durch die Vielzahl der Streuvorgänge statistisch verteilt. Mittels der zeitaufgelösten und der RF-modulierten Spektroskopie ist es möglich, die statistisch verteilte mittlere optische Weglänge im streuenden Medium zu messen bzw. dem jeweiligen Absorptionswert zuzuordnen.

Eine neue Entwicklung auf diesem Gebiet ist beschrieben in: A. Duncan et al. "A multiwavelength, wide band, intensity modulated optical spectrometer for near infrared spectroscopy and imaging", Proc. SPIE 1888 (1993), 248-257.

Die in den vorstehenden Publikationen beschriebenen Verfahren zur Bestimmung der Laufzeit bzw. der Phasenverschiebung als Laufzeit-Parameter eignen sich auch für die vorliegende Erfindung. Hinsichtlich der Meßtechnik kann deswegen im wesentlichen auf die bekannten und publizierten Verfahren Bezug genommen werden. Die Erfindung unterscheidet sich jedoch vor allem dadurch grundlegend von der zeitaufgelösten Spektroskopie, daß sie nicht auf der wellenlängenabhängigen Absorption des Analyts basiert, d.h. es handelt sich nicht um ein spektroskopisches Verfahren. Wie nachfolgend dargelegt wird, ist die Absorption durch die Glucose selbst in optimalen Wellenlängen-Bereichen so gering, daß sie die Laufzeit des Lichts in einer biologischen Matrix nicht meßbar beeinflußt. Darüber hinaus wird vorzugsweise außerhalb der unter spektroskopischen Gesichtspunkten scheinbar optimalen Wellenlängenbereiche gemessen. Die bei spektroskopischen Verfahren erforderliche Messung bei mindestens zwei scharf definierten Wellenlängen in einem breiten Spektralbereich ist bei der Erfindung nicht erforderlich. Vielmehr genügt eine Messung bei einer Wellenlänge ohne aufwendige Maßnahmen zur schmalbandigen Selektion (z.B. Filterung) des Primär- oder des Sekundärlichts.

Im Vergleich zu vorbekannten Verfahren zur nicht-invasiven reagenzienfreien Glucosebestimmung, insbesondere den Versuchen, die Glucosekonzentration in streuenden biologischen Matrices spektroskopisch zu bestimmen, bietet die Erfindung vor allem folgende Vorteile:

- Die Bestimmung der Laufzeit-Parameter ist weitgehend unabhängig von der absoluten Lichtintensität des detektierten Sekundärlichts. Dadurch wirken sich Schwankungen in der optischen Ankopplung des Lichtsenders bzw. des Lichtempfängers an die Grenzfläche der Matrix auf die Meßgenauigkeit praktisch nicht aus.

- Die Meßgenauigkeit wird weniger durch Änderungen der optischen Absorption von stark absorbierenden Störsubstanzen, wie insbesondere des roten Blutfarbstoffes und des Wassers beeinflußt. Diese Störungen sind von entscheidender Bedeutung bei spektroskopischen Verfahren. Beispielsweise führen bereits kleine Schwankungen des Blutvolumens in einem untersuchten Gewebe zu so starken Schwankungen der gemessenen Sekundärintensität, daß dieses Störsignal um mehrere Größenordnungen größer als das spektroskopische Nutzsignal ist. Zwar sind auch die Laufzeit-Parameter nicht völlig unabhängig von Änderungen der optischen Absorption in der Matrix, jedoch ist der Störeinfluß geringer. Außerdem kann die Wellenlänge des Primärlichts in Spektralbereiche gelegt werden, in denen die Absorption der Störsubstanzen verhältnismäßig gering und vor allem konstant ist.

- Die Abhängigkeit der Laufzeit von der Glucosekonzentration ist so groß, daß schon mit relativ kurzen Meßabständen zwischen Einstrahlungsort und Detektionsort von weniger als 4 cm (vorzugsweise höchstens 3 cm, besonders bevorzugt höchstens 2 cm) in einem Hautgewebe eine ausreichende Meßgenauigkeit erreichbar ist. Dies ist von großer Bedeutung, weil einerseits bei größeren Meßabständen die Intensität des Sekundärlichts so gering wird, daß es mit vertretbarem meßtechnischem Aufwand nicht mehr detektiert werden kann und weil andererseits in der Haut die Glucosekonzentration vorzugsweise dicht unter der Hautoberfläche gemessen werden sollte. Je größer der Meßabstand ist, desto mehr konzentriert sich die Messung auf tiefere Gewebeschichten.

Die Erfindung wird im folgenden anhand von in den Figuren dargestellten Ausführungsbeispielen näher erläutert; es zeigen:

Fig. 1    Ein Absorptionsspektrum von Glucose in Wasser in einem ersten Wellenlängenbereich für unterschiedliche Glucosekonzentrationen;

Fig. 2    ein Differenz-Absorptionsspektrum von Glucose in Wasser gegen reines Wasser in einem zweiten Wellenlängenbereich für unterschiedliche Glucosekonzentrationen;

Fig. 3    eine Prinzipdarstellung einer ersten Ausführungsform der Erfindung, bei der die Laufzeit des Lichts direkt bestimmt wird;

Fig. 4    eine graphische Darstellung eines mit einer Vorrichtung gemäß Figur 3 gemessenen Sekundärlicht-Impulses;

Fig. 5    eine graphische Darstellung von einer mit einer Vorrichtung gemäß Figur 3 gemessenen Korrelation zwischen Laufzeit und Glucose-Konzentration;

Fig. 6 ein Blockschaltbild zur Erläuterung einer zweiten Ausführungsform der Erfindung, bei der die Phasenverschiebung des Lichts bestimmt wird;

Fig. 7 eine graphische Darstellung von einer mit einer Vorrichtung gemäß Fig. 6 gemessenen Korrelation zwischen Phasenverschiebung und Glucose-Konzentration;

Fig. 8 eine graphische Darstellung von Meßergebnissen an Gewebe mit dem erfindungsgemäßen Verfahren im Vergleich zu einem konventionellen Verfahren;

Fig. 9 eine Prinzipdarstellung der Anordnung von Einstrahlungs- und Detektionsorten bei einer weiteren Ausführungsform der Erfindung zur Bestimmung der Phasenverschiebung als Laufzeit-Parameter;

Fig. 10 eine schematische Aufsicht auf eine biologische Matrix zur Verdeutlichung der Positionierung von zwei Einstrahlungsorten und einem Detektionsort bei einer weiteren bevorzugten Ausführungsform der Erfindung in Verbindung mit einer graphischen Darstellung zur Verdeutlichung des dabei erzielten Effektes;

Fig. 11 ein Blockschaltbild einer weiteren Ausführungsform der Erfindung;

Fig. 12 ein Blockschaltbild einer bevorzugten Ausführungsform der Frequenzstabilisierung für zwei Frequenzgeneratoren zur Erzeugung einer konstanten Differenzfrequenz.

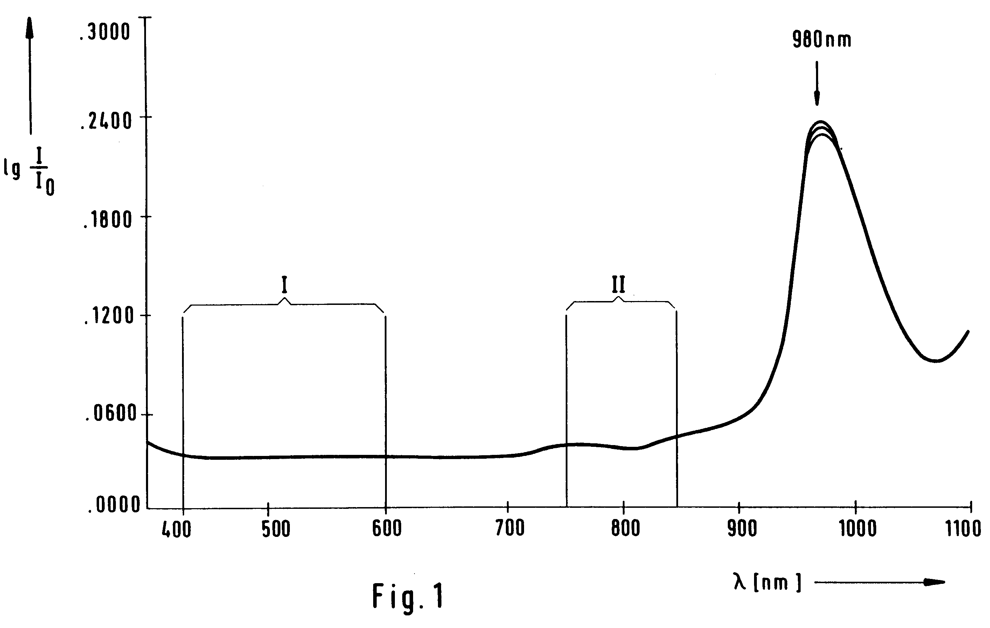

Figur 1 zeigt ein Absorptionsspektrum von Glucose in Wasser. Aufgetragen ist dabei der dekadische Logarithmus der Relation aus gemessener Intensität und eingestrahlter Intensität ($\lg I/I_0$) in Transmission für eine Küvettendicke von 1 cm und vier Glucosekonzentrationen, nämlich 0, 1, 5 und 10%. Man erkennt, daß sich die Spektren für diese vier Konzentrationen nur in einem kleinen Wellenlängenbereich bei etwa 980 nm geringfügig unterscheiden. Der maximale Signalunterschied zwischen dem Meßwert von reinem Wasser und der 10%-igen Glucoselösung bei dieser Wellenlänge ist kleiner als 2 %. Bei anderen Wellenlängen ist der Unterschied noch wesentlich geringer. Dabei ist die in dem Experiment verwendete Variation der Glucosekonzentration sehr viel größer als die reale physiologische Glucosekonzentration. Bezogen auf eine realistische Glucoseänderung im physiologischen Bereich von 100 mg/dl entspricht die Änderung bei 980 nm weniger als 0,02 %. Die Änderung dI/dC des Meßsignals I in Abhängigkeit von der Glucosekonzentration C wird nachfolgend als "relativer Signalhub (relativ signal change)" bezeichnet und quantitativ in % je 100 mg/dl Änderung der Glucosekonzentration ausgedrückt.

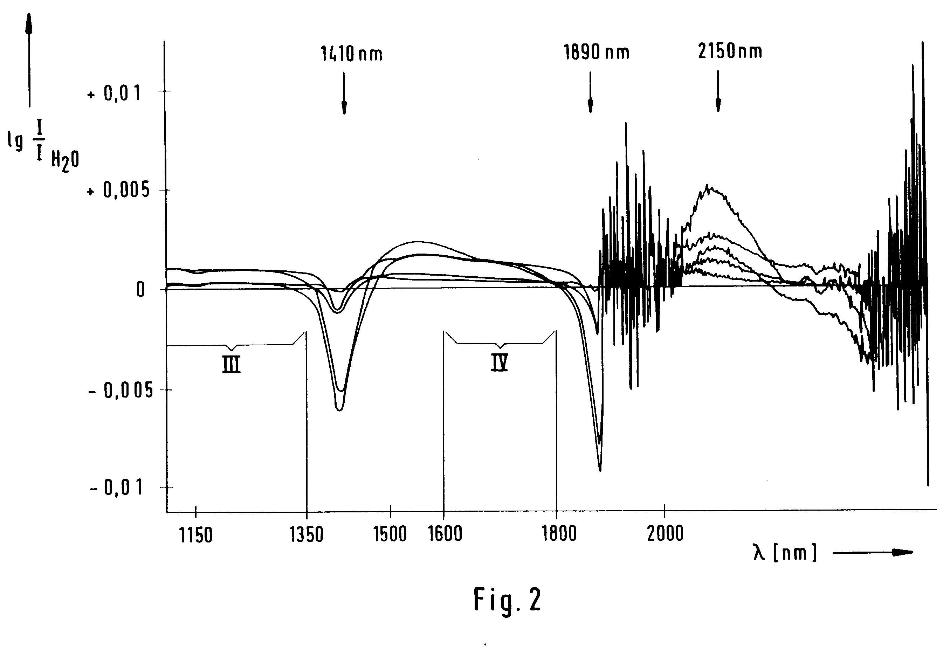

Figur 2 zeigt ein ähnliches Spektrum im anschließenden Wellenlängenbereich zwischen etwa 1100 nm und 2500 nm. Es handelt sich dabei um ein Differenzspektrum für Glucosekonzentrationen zwischen 0 und 600 mg/dl gegen reines Wasser. Negative Werte bedeuten dabei eine im Vergleich zum reinen Wasser verringerte Absorption. Dabei beträgt die maximale nutzbare Änderung der Signalintensität insgesamt weniger als 0,3 %, also im Mittel weniger als 0,05 % je 100 mg/dl Änderung der Glucosekonzentration.

Die Figuren 1 und 2 zeigen insgesamt, daß über weite Spektralbereiche die Abhängigkeit der optischen Absorption von der Glucosekonzentration extrem gering ist. Als Spektralbereiche mit einer "geringen" Abhängigkeit der Absorption von der Glucosekonzentration werden die Wellenlängenbereiche angesehen, in denen der relative Signalhub dI/dC bei einer Transmissionsmessung an einer klaren Glucoselösung weniger als 0,01 % je 100 mg/dl Änderung der Glucosekonzentration beträgt. Aufgrund der in den Figuren 1 und 2 dargestellten Meßergebnisse könnten in diesem Spektralbereich am ehesten die Wellenlängen um etwa 980 nm, 1410 nm, 1890 nm und 2150 nm zur spektroskopischen Analyse der Glucose geeignet sein.

Aus den Meßdaten der Figuren 1 und 2 läßt sich ableiten, daß selbst bei den "spektroskopisch optimalen" Wellenlängen die Absorption einer wässrigen Glucoselösung eine so geringe Abhängigkeit von der Glucosekonzentration zeigt, daß durch solche Absorptionsänderungen verursachte Änderungen der Laufzeit des Lichtes um mehrere Größenordnungen zu gering wären, um praktisch meßbar zu sein. Dies belegt, daß die im Rahmen der Erfindung festgestellte Korrelation zwischen der Laufzeit des Lichts in einer biologischen Matrix und der Glucosekonzentration keinesfalls mit der Absorption durch die Glucose erklärt werden kann.

Im Rahmen der vorliegenden Erfindung sind die in den Figuren 1 und 2 mit römischen Ziffern bezeichneten Wellenlängenbereiche mit geringer Absorption einer wässrigen Glucose-Lösung besonders bevorzugt, nämlich:

I. 400 bis 600 nm
II. 750 bis 850 nm, vorzugsweise 780 bis 825 nm, besonders bevorzugt 800 bis 805 nm
III. 1050 bis 1350 nm, vorzugsweise zwischen 1200 und 1300 nm und
IV. 1600 bis 1800 nm, vorzugsweise 1630 bis 1770 nm, besonders bevorzugt 1630 nm bis 1670 nm oder 1730 nm bis 1770 nm.

Das Primärlicht sollte bevorzugt näherungsweise monochromatisch sein. "Monochromatisch" ist dabei im praktischen Sinne dahingehend zu verstehen, daß der überwiegende Teil der Intensität in einem definierten Wellenlängenbereich mit einem ausgeprägten Maximum emittiert wird, bzw. die Messung in einem entsprechend begrenzten Wellenlängenbereich erfolgt. Die Halbwertsbreite sollte weniger als 100 nm, bevorzugt weniger als 50 nm betragen. Im Gegensatz zu den spektroskopischen Verfahren zur nicht-invasiven Glucose-Analyse können relativ breitbandige Lichtquellen (mit Halbwertsbreiten größer als 20 nm) wie beispielsweise Leuchtdioden oder andere Halbleiter-Lichtquellen ohne anschließende spektrale Selektion eingesetzt werden. Hierdurch werden die Kosten für die Apparatur erheblich verringert. Soweit hier auf "die Wellenlänge" der Lichtquelle bzw. des Primärlichts Bezug genommen wird, bezieht sich diese Aussage auf die Wellenlänge des Intensitätsmaximums.

Im Gegensatz zu den bekannten spektroskopischen Verfahren ist es ausreichend, wenn der Detektionsschritt bei jeweils nur einer Wellenlänge durchgeführt wird. Die Tatsache, daß das Meßsignal von der Wellenlänge weitgehend unabhängig ist, ermöglicht es, für die Messung solche Wellenlängenbereiche auszuwählen, bei denen die Störungen durch stark absorbierende Substanzen möglichst gering sind. In dem Bereich um 802 nm ist die gemessene Intensität näherungsweise unabhängig von dem Konzentrationsverhältnis zwischen Hb und $HbO_2$, weil diese Substanzen dort einen isosbestischen Punkt besitzen. Dies gilt auch in einem breiten isosbestischen Bereich zwischen 1200 und 1300 nm. Zusätzlich ist in diesem Bereich die Absorption von Hämoglobin und Wasser etwa gleich groß. Dadurch ergibt sich eine besonders gute Unabhängigkeit der gemessenen Intensität von dem Verhältnis von Hb, $HbO_2$ und $H_2O$.

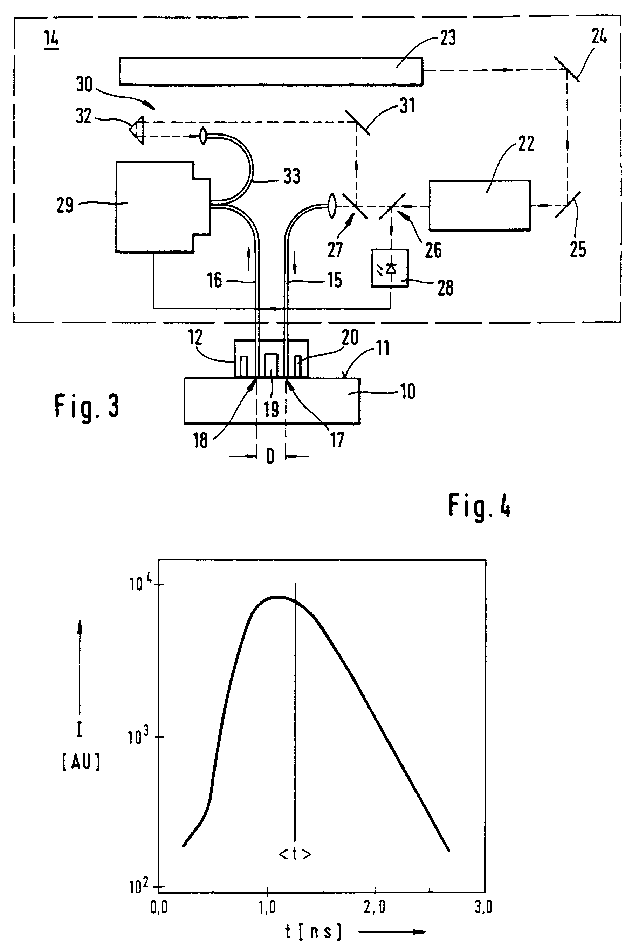

Im Rahmen der experimentellen Erprobung der Erfindung hat sich gezeigt, daß verhältnismäßig kurze Wellenlängen, insbesondere zwischen 400 und 600 nm wegen der damit verbundenen verhältnismäßig geringen Eindringtiefe in biologisches Gewebe besonders vorteilhaft sein können. Aus experimentellen Beobachtungen bestehen Anhaltspunkte, daß dies insbesondere wegen der gleichmäßigeren Verteilung des Blutes in den obersten Hautschichten und möglicherweise auch wegen einer besseren Korrelation der Glucosekonzentration mit der Blutglucose vorteilhaft ist. Figur 3 zeigt schematisiert einen experimentellen Aufbau zur Messung der Laufzeit an einer lediglich symbolisch als Rechteck dargestellten biologischen Matrix 10. In Kontakt mit einer Grenzfläche 11 der biologischen Matrix 10 befindet sich ein Meßkopf 12. Die biologische Matrix 10 ist vorzugsweise Hautgewebe, insbesondere an der Fingerbeere, der Oberbauchdecke, dem Nagelbett, der Lippe, der Zunge oder dem inneren Oberarm des Menschen, wobei in diesem Fall die Grenzfläche 11 von der Hautoberfläche gebildet wird. Auch am Gewebe der Skleren kann eine erfindungsgemäße Analyse vorteilhaft durchgeführt werden.

Von einer insgesamt mit 14 bezeichneten Laufzeit-Meßeinrichtung führen Glasfaser-Lichtleitkabel 15 und 16 zu dem Meßkopf 12. Das eine Lichtleitkabel 15 dient zum Einstrahlen von Licht in die biologische Matrix an einem Einstrahlungsort 17 und wird als Sender-Lichtleitkabel bezeichnet, während das andere Lichtleitkabel 16 zur Detektion von an einem Detektionsort 18 austretendem Licht dient und deswegen als Detektions-Lichtleitkabel bezeichnet wird.

Der Meßkopf 12 enthält weiterhin Mittel, um die Temperatur der biologischen Matrix 10 in dem Meßbereich zwischen dem Einstrahlungsort 17 und dem Detektionsort zu messen und gemäß einer weiteren bevorzugten Ausführungsform konstant zu halten. Symbolisch in Figur 3 dargestellt sind zu diesem Zweck ein zentral angeordneter IR-Temperatursensor 19 und ein ringförmiger Widerstands-Heizleiter 20. Die Heizleistung des Heizleiters 20 wird mit einer nicht dargestellten Regelung so geregelt, daß die Temperatur in dem Meßbereich konstant gehalten wird.

Die Laufzeit-Meßeinrichtung besteht im wesentlichen aus einem modengekoppelten Festkörperlaser als Lichtquelle und einer Streak-Kamera als Detektor.

Der Festkörper-Laser 22 ist im dargestellten Fall ein Titan-Saphir-Laser, der von einem Argon-Gas-Laser 23 über zwei Umlenkspiegel 24 und 25 optisch gepumpt wird. Man erhält dabei kurze Lichtpulse mit einer Impulsdauer von etwa 2 ps ($2 \cdot 10^{-12}$ sec) und einer Wiederholungsrate von 82 MHz. Die Wellenlänge des Lichts war bei dem Versuchsaufbau zwischen 740 und 900 nm, also im nahen Infrarot-Bereich, durchstimmbar. Die mittlere Ausgangsleistung des verwendeten Festkörper-Lasers lag bei ca. 1 W.

Das Licht des Festkörper-Lasers 22 wird über zwei Strahlteiler 26,27 in das Sender-Lichtleitkabel 15 eingekoppelt. Durch den ersten Strahlteiler 26 wird ein Teil des Lichts auf eine Photodiode 28 gelenkt, deren Signal zur Synchronisation der als Detektor verwendeten Streak-Kamera 29 dient.

Von dem zweiten Strahlteiler 27 wird ein weiterer Teilstrahl zu einer Verzögerungsstrecke 30 abgelenkt, die im dargestellten Fall aus einem weiteren Umlenkspiegel 31, einem 180°-Reflexionsprisma 32 und einem Glasfaser-Lichtleitkabel 33 zur Verbindung mit der Streak-Kamera 29 besteht. Somit wird sowohl das über die Lichtleitkabel 15 und 16 durch die biologische Matrix 10 geleitete Licht als auch das über die Verzögerungsstrecke 30 geleitete Licht in gleicher Weise von der Streak-Kamera 29 gemessen.

Vor der eigentlichen Analysemessung erfolgt eine Justierung der Zeitachse der Laufzeit-Meßeinrichtung 14. Dazu werden die Enden der Glasfaser-Lichtleitkabel 15,16 aus dem Meßkopf 12 herausgenommen und dicht zusammengebracht. Dieser Zustand entspricht einer Laufzeit t=0. Bei der eigentlichen Messung befinden sich die Enden der Lichtleitkabel 15 und 16 an dem Einstrahlungsort bzw. dem Detektionsort 18 in einem definierten Meßabstand D auf der Grenzfläche 11 der biologischen Matrix 10. In diesem Zustand wird die Verzögerungsstrecke 30 so lange verändert, bis in dem zeitlichen Abbildungsfenster der Streak-Kamera sowohl das über die Verzögerungsstrecke 30 laufende Referenzsignal als auch das über die Kabel 15,16 laufende Meßsignal erfaßt wird. Auf diese Weise kann die Laufzeit zwischen Einstrahlungsort 17 und Detektionsort 18 mit einer Genauigkeit von wenigen ps gemessen werden.

Ein typisches Ergebnis einer solchen Messung ist in Figur 4 dargestellt. Es zeigt den zeitlichen Verlauf der an dem Detektionsort 18 gemessenen Lichtintensität, der aus einem Impuls des Festkörper-Lasers 22 resultiert. Man erkennt, daß der kurze Delta-Impuls des Primärlichts durch die Mehrfachstreuung in der biologischen Matrix 10 und die dadurch mögliche Vielzahl von verschieden langen Wegen von dem Einstrahlungsort zu dem Detektionsort 18 stark verbreitert ist. Die Halbwertsbreite des dargestellten Sekundärlicht-Impulses beträgt ca. 1 ns.

Aus dem Verlauf des Sekundärlicht-Impulses läßt sich die mittlere Laufzeit $\langle t \rangle$ des Lichts zwischen Einstrahlungsort 17 und Detektionsort 18 berechnen, die der mittleren optischen Weglänge zwischen diesen beiden Orten entspricht. In Figur 4 ist diese mittlere Laufzeit eingezeichnet. Sie beträgt in diesem Fall ca. 1,2 nsec.

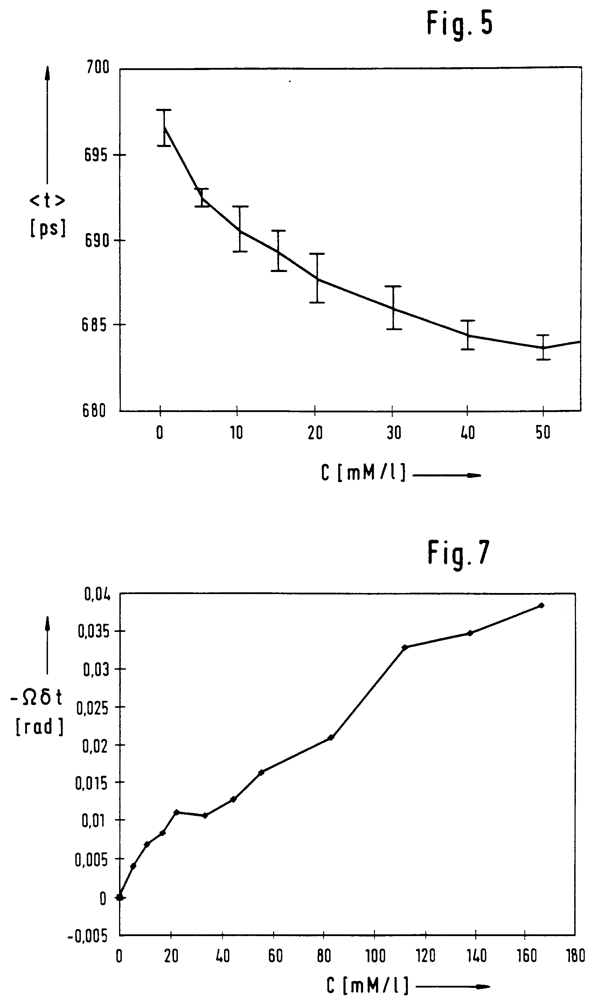

Figur 5 zeigt die Abhängigkeit der mittleren Laufzeit $\langle t \rangle$ in ps von der Glucosekonzentration in mmol. Dabei wurde als Experimentalmodell einer biologischen Matrix eine Suspension von Latex-Partikeln (Durchmesser ca. 2,5 µm) in Wasser verwendet, der ein IR-Farbstoff zugesetzt wurde. Die Konzentration der Partikel und des Farbstoffs wurden dabei so gewählt, daß Absorptions- und Streukoeffizient etwa den für Gewebe typischen Werten entsprachen. Der Meßabstand D zwischen Einstrahlungsort 17 und Detektionsort 18 betrug ca. 15 mm.

Figur 5 zeigt deutlich, daß die mittlere Laufzeit $\langle t \rangle$ mit zunehmender Glucosekonzentration abnimmt. Obwohl dieser Effekt verhältnismäßig klein ist, kann er mit guter Genauigkeit bestimmt werden. Dabei ist im Vergleich zur invivo-Analyseverfahren für Glucose, die auf der Messung einer Lichtintensität basieren, ein besonderer Vorteil darin zu sehen, daß der Meßparameter Laufzeit eine wesentlich geringere Abhängigkeit von einer reproduzierbaren Einkopplung bzw. Auskopplung des Lichts am Einstrahlungsort- bzw. Auskopplung des Lichts am Einstrahlungs- bzw. Detektionsort zeigt.

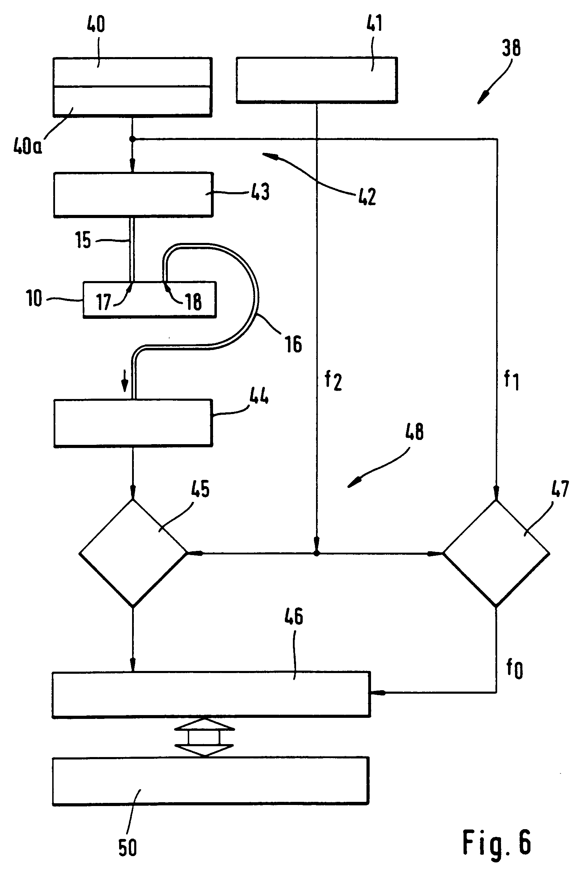

Figur 6 zeigt beispielhaft als Blockschaltbild den Aufbau einer Einrichtung 38 zur Messung der Phasenverschiebung (Phasenmeßeinrichtung). Das Meßprinzip (Heterodyn-Verfahren) wird auch bei der Phasenfluorometrie angewendet.

Ein Frequenzgenerator 40 erzeugt eine hochfrequente periodische Schwingung, mit der über eine Verstärkerschaltung (Treiberstufe) 40a ein Lichtsender 43, beispielsweise eine Leuchtdiode, angesteuert wird. Dadurch wird das von dem Lichtsender 43 ausgehende Primärlicht mit einer hochfrequenten Frequenz $f_1$ moduliert, die als Trägerfrequenz bezeichnet wird. Die Frequenz sollte mehr als 50 MHz, vorzugsweise mehr als 200 MHz, besonders bevorzugt mehr als 500 MHz betragen. Das Licht des Lichtsenders 43 wird ebenso wie bei der Ausführungsform gemäß Figur 3 über ein erstes Glasfaser-Lichtleitkabel 15 an einem Einstrahlungsort 17 als Primärlicht in die biologische Matrix 10 eingestrahlt. Die durch die Bauteile 40, 40a, 43 und 15 gebildeten Lichteinstrahlungsmittel 42 können auch in anderer Weise realisiert sein. Diverse Möglichkeiten sind aus den eingangs diskutierten und zahlreichen weiteren Publikationen bekannt.

Das an einem Detektionsort 18 aus der biologischen Matrix 10 austretende Licht wird mit Detektionsmitteln 48 detektiert, die bei der dargestellten Ausführungsform ein Detektionslichtleitkabel 16 einschließen, durch das das Sekundärlicht an einen Lichtempfänger (Detektor) 44 übertragen wird. Der Lichtempfänger erzeugt ein der Lichtintensität entsprechendes elektrisches Ausgangssignal, das einem Signalmischer 45 zugeführt wird. An dem Signalmischer 45 liegt gleichzeitig das Ausgangssignal eines zweiten Frequenzgenerators 41 an, der mit dem Frequenzgenerator 40 synchronisiert ist und dessen Ausgangsfrequenz $f_2$ sich nur geringfügig (beispielsweise um $f_0 = 1$ kHz) von der Frequenz $f_1$ des ersten Frequenzgenerators 40 unterscheidet. Die Differenz-Frequenz $f_0$ ist so gewählt, daß sie einen Lock-in-Verstärker problemlos synchronisiert und das Eigenrauschen der erforderlichen Vorverstärker gering ist.

Das Ausgangssignal des Mischer 45 liegt am Eingang eines Lock-in-Verstärkers 46 an. Als Referenzsignal erhält der Lock-in-Verstärker 46 das unmittelbare Mischsignal der Frequenzgeneratoren 40 und 41, deren Ausgangssignale über einen zweiten Mischer 47 gemischt werden.

Der Lock-in-Verstärker 46 erzeugt zwei Ausgangssignale, die dem Realteil bzw. dem Imaginärteil des periodischen Meßsignals entsprechen. Daraus lassen sich die Phasendifferenz, die AC-Amplitude und die DC-Amplitude, jeweils bezogen auf das Referenzsignal, berechnen. Die Phasenmeßeinrichtung kann auch in anderer Weise realisiert sein. Zahlreiche Varianten elektronischer Schaltungen zur Messung einer solchen Phasenverschiebung sind bekannt und können auch bei der vorliegenden Erfindung eingesetzt werden. Insofern wird insbesondere auf die weiter oben genannten Publikationen zur Phasenfluorometrie und zur zeitaufgelösten Spektroskopie Bezug genommen.

Die Phasenverschiebung des an dem Detektionsort 18 gemessenen Sekundärlichts in Relation zu dem an dem Einstrahlungsort 17 eingestrahlten Primärlicht ist ein Maß für die Laufzeit des Lichts in der biologischen Matrix und somit ein Maß für die Glucosekonzentration. Zur Durchführung des zur Berechnung der Glucosekonzentration erforderlichen Auswerteschrittes (d.h. zur Ausführung eines Auswertealgorithmus) sind Auswertemittel 50, zweckmäßigerweise in Form eines Mikrocomputers, vorgesehen.

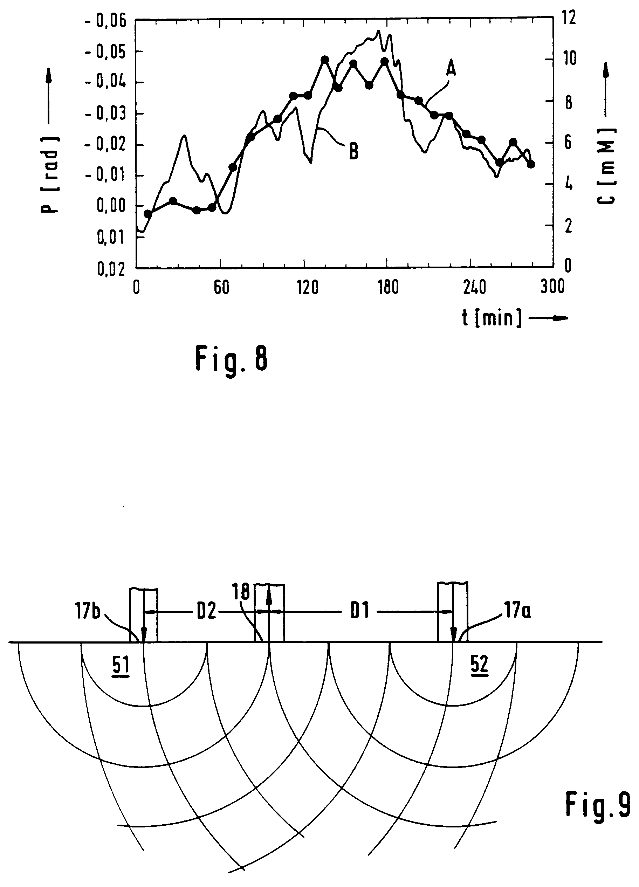

Figur 7 zeigt Meßergebnisse, die mit einer Meßapparatur mit dem prinzipiellen Aufbau gemäß Figur 6 gewonnen wurden. Untersucht wurde eine Suspension von Fetttröpfchen in Wasser (Intralipid), welche sich in einer Küvette mit einer Dicke von 25 mm befand. Die Messung erfolgte in Transmission, wobei die Lichtleitfasern für die Einstrahlung des Primärlichts und die Detektion des Sekundärlichts auf gegenüberliegenden Seiten der Küvette unmittelbar auf die Küvettenwand aufgesetzt wurden.

Dargestellt ist das Verhalten der Phasenverschiebung als Funktion der Glucosekonzentration für eine Meßwellenlänge L=690 nm. Die Phasenverschiebung entspricht dem negativen Produkt aus der Kreisfrequenz der Modulation $\Omega$ und der Änderung der Laufzeit $\delta t$ und ist deshalb als $-\Omega \delta t$ bezeichnet. Die in Figur 7 dargestellte Zunahme dieser Meßgröße entspricht somit einer Abnahme der Laufzeit, steht also im Einklang mit dem in Figur 5 dargestellten Meßergebnis.

Figur 8 zeigt Ergebnisse der Analyse von Glucose in Gewebe mit Hilfe des erfindungsgemäßen Verfahrens. Dargestellt ist einerseits der Verlauf der Glucosekonzentration C (rechte Ordinate, schwarze Punkte, Kurve A) und andererseits der Verlauf der Phasenverschiebung P (linke Ordinate, Kurve B) über die Zeit t in Minuten. Dabei wurden die Meßwerte der Kurve A konventionell invasiv durch Entnahme von Blutproben und Analyse mit einem Teststreifen-Analysesystem bestimmt, während die Kurve B mit dem erfindungsgemäßen Verfahren gemessen wurde. Man erkennt, daß das erfindungsgemäße Verfahren mit den konventionellen Messungen gut korreliert. Durch zeitliche Mittelwertbildung können die in Figur 8 erkennbaren kurzzeitigen Schwankungen reduziert werden.

Zur Bestimmung der absoluten Glucosekonzentration ist bei jeder Ausführungsform der Erfindung eine Kalibration erforderlich, bei der gleichartige Detektionsschritte an einer biologischen Matrix mit bekannter Glucosekonzentration ausgeführt werden. Durch solche Kalibrationsmessungen gewinnt man in üblicher Weise eine Kalibrationskurve, die die funktionale Abhängigkeit der Glucosekonzentration von dem Laufzeit-Parameter beschreibt. Bei einem geraden Verlauf der Kalibrationskurve sind im Regelfall zwei Kalibrations-Detektionsmessungen bei zwei unterschiedlichen Glucosekonzentrationen erforderlich. Bei einem gekrümmten Verlauf wird eine größere Anzahl von Kalibrationsmessungen durchgeführt.

Ein Vorteil der Phasenverschiebung (als "indirekter" Laufzeit-Parameter) im Vergleich zu der unmittelbaren Bestimmung der Laufzeit liegt, wie erwähnt, darin, daß mit einem wesentlich geringeren meßtechnischen Aufwand eine ausreichende Genauigkeit erreicht werden kann.

Im Rahmen der vorliegenden Erfindung wird vorzugsweise zusätzlich zu der Phasenverschiebung die Wechselstromamplitude (AC-Amplitude) des Sekundärlichts bestimmt und in dem Auswerteschritt zur Ermittlung der Glucosekonzentration verwendet. Die Messung der AC-Amplitude des Sekundärlichts (in Relation zu der AC-Amplitude des Primärlichts) ist leicht in Kombination mit der Phasenverschiebung möglich. Dies wurde im Zusammenhang mit Figur 6 beschrieben und gilt auch für andere bekannte Meßverfahren.

Weiterhin kann auch die Größe des Gleichstrom-Signalanteils (d.h. die DC-Amplitude) des Sekundärlichts in Relation zu der DC-Amplitude des Primärlichts bei solchen Messungen bestimmt werden. Gemäß einer weiteren bevorzugten Ausführungsform der Erfindung wird die gemessene DC-Komponente des Sekundärlichts (in Relation zu der DC-Komponente des Primärlichts) in dem Auswerteschritt zusätzlich zu der Phasenverschiebung zur Bestimmung der Glucosekonzentration verwendet.

Wenn eine Mehrzahl von Meßgrößen, wie beispielsweise Phasenverschiebung und AC oder Phasenverschiebung und DC verwendet werden, um einen einzigen Konzentrationswert der Glucose zu bestimmen, muß selbstverständlich in dem Auswerteschritt ein Algorithmus verwendet werden, der geeignet ist, eine Mehrzahl von Eingangsvariablen (Phasenverschiebung, AC, DC) mit einer Ausgangsvariablen (Glucosekonzentration C) zu verknüpfen.

Im einfachsten Fall kann ein solcher Algorithmus eine fest vorgegebene mathematische Operation einschließen, durch die aus den gemessenen Eingangsvariablen ein Zwischenwert abgeleitet wird. Der Zwischenwert kann als Meßergebnis R bezeichnet werden. Dieses Meßergebnis R kann dann in der üblichen Weise durch Kalibration mit der Glucosekonzentration C verknüpft werden, wobei mindestens zwei, vorzugsweise jedoch mehrere Standardproben mit bekannter Glucosekonzentration gemessen werden.

In jüngerer Zeit werden in zunehmendem Umfang mathematisch aufwendigere Verfahren eingesetzt, um in der Analysetechnik die Korrelation zwischen den Meßgrößen (Eingangsvariablen) und der gesuchten Konzentration (Ausgangsvariablen) zu verbessern und dadurch eine höhere Analysegenauigkeit zu erreichen. Hierzu gehören iterative Methoden zur optimalen Beschreibung der Relation von Eingangsvariablen und Ausgangsvariablen. Multilineare und nichtlineare Algorithmen können verwendet werden, um mehrere Faktoren miteinander zu verknüpfen, die für die Auswertung der analytischen Messungen erforderlich sind. Diese Vorgehensweise ist auch bei der vorliegenden Erfindung möglich und kann zur Verknüpfung der Meßgrößen Phasenverschiebung, AC und DC mit der Konzentration verwendet werden. Daneben ermöglichen diese Verfahren, weitere Einflußfaktoren (wie beispielsweise die Temperatur am Meßort) zusätzlich zu berücksichtigen.

Wie erwähnt, kann es besonders bevorzugt sein, mit einer im Vergleich zur vorbekannten Verfahren außerordentlich hohen Frequenz von mehr als 500 MHz zu messen. Dies ist im Rahmen der Erfindung vor allem deshalb vorteilhaft, weil dadurch eine geringe Eindringtiefe des Lichtweges zwischen Einstrahlungsort und Detektionsort erreicht wird und somit gezielt die Glucosekonzentration in den obersten Hautschichten gemessen werden kann.

Zur Verbesserung der Genauigkeit kann es zweckmäßig sein, das Primärlicht mit mehreren unterschiedlichen Trägerfrequenzen zu modulieren und dabei jeweils die Phasenverschiebung als Laufzeit-Parameter zu bestimmen.

Die geometrischen Bedingungen hinsichtlich der Abmessungen des Einstrahlungsortes 17 und des Detektionsortes 18, hinsichtlich der Anordnung dieser Orte auf der Grenzfläche 11 der biologischen Matrix 10 und insbesondere hinsichtlich des Meßabstandes D können im Einzelfall empirisch festgelegt werden. Dabei sind folgende Grundregeln zu beachten.

Die Begriffe "Einstrahlungsort" und "Detektionsort" sind geometrisch zu verstehen, nämlich als der Teilbereich der Grenzfläche einer biologischen Matrix, an dem Lichtstrahlen, die bei dem jeweiligen Detektionsschritt für den ermittelten Laufzeit-Parameter bestimmend sind, durch die Grenzfläche hindurchtreten. Soweit nachfolgend Angaben über Distanzen zwischen Einstrahlungsort und Detektionsort gemacht werden, beziehen sich diese auf die Mitte des jeweiligen Einstrahlungsortes bzw. Detektionsortes, jeweils betrachtet in Richtung des Abstandes (d.h. der kürzesten Verbindung) zwischen Einstrahlungsort und Detektionsort.

Um die Laufzeit mit guter Auflösung einem bestimmten Meßabstand zuordnen zu können, sollte die Dimension des Einstrahlungsortes und des Detektionsortes in Richtung des genannten Abstandes nicht zu groß sein. Vorzugsweise beträgt sie weniger als 2 mm, besonders bevorzugt weniger als 1 mm.

Um die für den erfindungsgemäßen Effekt erforderliche Vielfachstreuung des Lichts in der biologischen Matrix zu gewährleisten, müssen der Einstrahlungsort und der Detektionsort relativ zueinander so angeordnet sein, daß das Licht auf dem Weg zwischen Einstrahlungsort und Detektionsort vielfach gestreut worden ist. Dies läßt sich durch eine ausreichende Länge des Meßabstandes gewährleisten. Typischerweise liegt die mittlere freie Weglänge von Photonen in Gewebe oder Blut zwischen etwa 0,01 mm und 0,1 mm. Der Lichtweg innerhalb der biologischen Matrix ist stets länger als die direkte Verbindung zwischen Einstrahlungsort und Detektionsort. Demzufolge ist schon bei einem Meßabstand von wenigen Millimetern die "Vielfachstreuungsbedingung" gewährleistet.

Um eine möglichst große Abhängigkeit des Meßsignals von der Glucosekonzentration zu erhalten, sollte der Meßabstand zwischen dem Einstrahlungsort und dem Detektionsort möglichst groß sein. Oberhalb einer im Einzelfall experimentell zu bestimmenden Grenze wird jedoch durch die abnehmende Intensität des Sekundärlichts das Signal/Rauschverhältnis schlecht. Vorzugsweise sollte deswegen der Meßabstand kleiner als 4 cm, bevorzugt kleiner als 3 cm, besonders bevorzugt kleiner als 2 cm sein. Diese Meßabstände sind deutlich kleiner als die Abstände, die im Rahmen der zeitaufgelösten Spektroskopie diskutiert werden. Auch hierdurch wird der in Relation zu bekannten Verfahren außerordentlich starke Effekt der vorliegenden Erfindung verdeutlicht. Obwohl bei der zeitaufgelösten Spektroskopie der Oximetrie-Parameter die Lichtabsorption des Analyten um mehrere Größenordnungen stärker ist, werden dort die großen Meßabstände für erforderlich gehalten, um eine ausreichend große Abhängigkeit der Phasenverschiebung von der Analyt-Konzentration zu erreichen. Die Tatsache, daß bei der erfindungsgemäßen Glucosebestimmung trotz der um mehrere Größenordnungen geringeren Absorption der Glucose das Meßsignal größer ist, zeigt, daß der vorliegenden Erfindung ein völlig anderer Wechselwirkungs-Mechanismus zwischen dem Licht und der biologischen Matrix zugrunde liegt.

Zur Optimierung der Meßgenauigkeit kann es besonders vorteilhaft sein, den mit der Glucosekonzentration korrelierenden Laufzeit-Parameter bei mehreren unterschiedlichen Meßabständen zwischen Einstrahlungsort und Detektionsort zu bestimmen. Die dadurch ermittelte funktionale Abhängigkeit der Laufzeit des Lichts (bei gleicher Wellenlänge) von dem Meßabstand zwischen Einstrahlungsort und Detektionsort kann in dem Auswerteschritt vorteilhaft zur Ermittlung der Glucosekonzentration verwendet werden.

Wenn diese funktionale Abhängigkeit von dem Meßabstand nicht nur für die Phasenverschiebung, sondern auch für andere Meßgrößen gemessen wird, wie insbesondere die AC- und/oder DC-Amplitude (des Sekundärlichts im Vergleich zu dem Primärlicht), kann eine erhebliche Menge an Meßdaten zur Korrelation mit der Glucosekonzentration verwendet werden. In solchen Fällen ist es besonders sinnvoll, die zuvor erwähnten numerischen mathematischen Methoden in dem Auswerteschritt einzusetzen. Insbesondere kann ein multivariater Regressionsalgorithmus, wie beispielsweise PLS oder ein lernfähiges (Computer-)System (neuronales Netz) verwendet werden.

Figur 9 dient zur Verdeutlichung einer weiteren bevorzugten Ausführungsform, bei der das Primärlicht in dem Detektionsschritt gleichzeitig an zwei verschiedenen Einstrahlungsorten 17a,17b eingestrahlt wird. Dabei überlagern sich die in der Figur symbolisch dargestellten in der biologischen Matrix propagierenden Lichtintensitätswellen 51,52. Bei geeigneter Wahl der Versuchsbedingungen, die im folgenden näher erläutert werden, kann mit einer solchen Anordnung an einem zwischen den Einstrahlungsorten 17a und 17b befindlichen Detektionsort 18 die Änderung einer Meßgröße des detektierten Lichtes festgestellt werden, die besonders empfindlich von Unterschieden der Laufzeit des Lichtes zwischen den beiden Einstrahlungsorten 17a,17b und dem dazwischen angeordneten Detektionsort 18 abhängt.

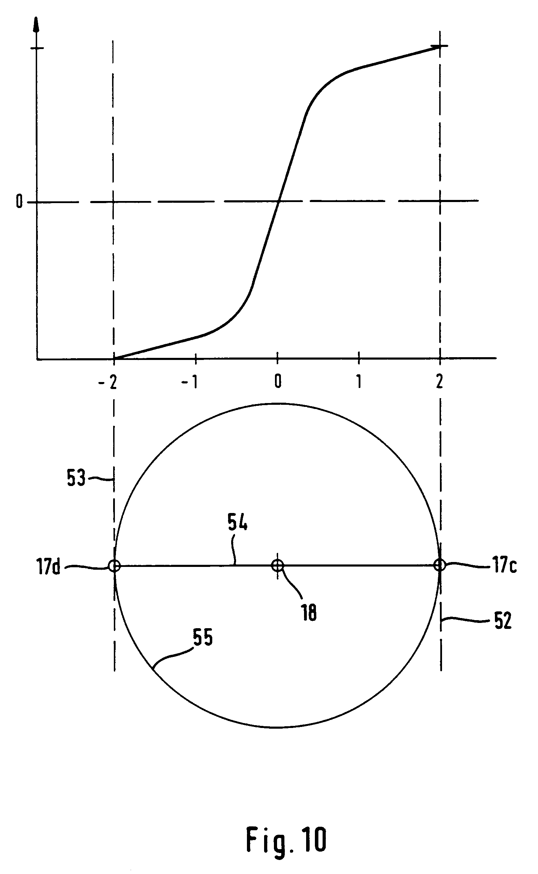

Der Effekt, der der bevorzugten Anordnung gemäß Figur 9 zugrunde liegt, wird anhand von Figur 10 erläutert. Zunächst muß klargestellt werden, daß es sich um eine Überlagerung von Lichtintensitätswellen und nicht etwa um die Interferenz von Lichtwellen handelt. Die Wellenlänge der Intensitätswellen entspricht dem Quotienten aus der Phasengeschwindigkeit der Welle im Medium und der Modulationsfrequenz und läßt sich auf etwa 0,1 m abschätzen.

Um die gewünschte hohe Nachweisempfindlichkeit zu erreichen, wird die Intensität, Modulationstiefe und Phasenlage des an den Einstrahlungsorten 17a und 17b eingestrahlten Primärlichts so aufeinander abgestimmt, daß in der Umgebung des Detektionsortes eine minimale AC- Amplitude und eine maximale Abhängigkeit des gemessenen Signals als Funktion einer Änderung des Detektionsortes zu beobachten ist. Figur 10 zeigt in der oberen Hälfte eine graphische Darstellung des aus der Überlagerung resultierenden AC-Signalanteils für den Fall, daß zwei Einstrahlungsorte in gleichen Meßabständen D von +2 cm bzw. -2 cm von einem in der Mitte dazwischen angeordneten Detektionsort 18 angeordnet sind. Eine solche Anordnung ist in der unteren Hälfte von Figur 10 dargestellt.

Bei gleichem Abstand und gleicher Intensität der Lichtquellen ist die genannte Bedingung erfüllt, wenn das Primärlicht an den Einstrahlungsorten 17c und 17d etwa gegenphasig (d.h. mit 180° Phasenverschiebung) moduliert wird.

Wenn sich bei einer solchen Anordnung der mittlere Lichtweg zwischen einem der Einstrahlungsorte und dem Detektionsort stärker ändert als zwischen dem anderen Einstrahlungsort und dem gleichen Detektionsort, so wird auch der Bereich des Phasensprungs verschoben. Diese Verschiebung läßt sich wesentlich empfindlicher als bei einer Anordnung mit nur einem Einstrahlungsort nachweisen, wenn sich der Detektionsort in dem steilen Bereich der Kurve gemäß Figur 10 befindet.

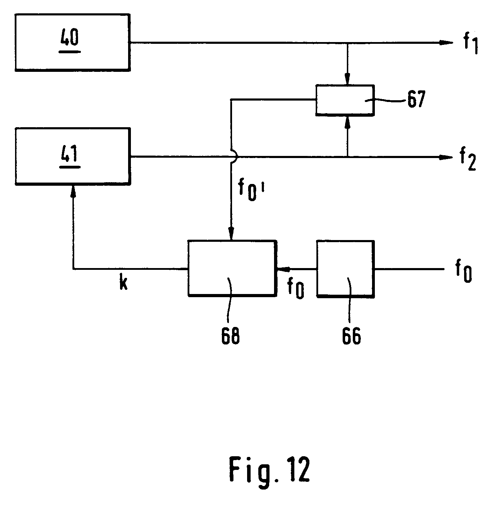

Zum Nachweis der Konzentration der Glucose läßt sich dieser Effekt nutzen, wenn Änderungen der Glucose-Konzentration den Lichtweg auf der einen Seite der Anordnung stärker beeinflussen als auf der anderen Seite. Diese Bedingung läßt sich dadurch erfüllen, daß sich der Meßabstand D1 zwischen dem ersten Einstrahlungsort und dem Detektionsort von dem Meßabstand D2 zwischen dem zweiten Einstrahlungsort und dem Detektionsort unterscheidet, wie dies in Figur 12 dargestellt ist. Alternativ kann man im Falle der Untersuchung eines Gewebes die Plazierung des Meßkopfes auf der Gewebeoberfläche (und somit der Einstrahlungsorte und des Detektionsortes) so wählen, daß auf dem Lichtweg zwischen dem ersten Einstrahlungsort und dem Detektionsort ein größeres Blutgefäß liegt, während sich zwischen dem zweiten Einstrahlungsort und dem Detektionsort kein vergleichbar großes Blutgefäß befindet. In diesem Fall kann auch mit gleichen Abständen zwischen Einstrahlungsort und Detektionsort auf beiden Seiten der Anordnung (wie in Figur 10 dargestellt) gearbeitet werden.

Zur Nutzung des anhand der Figuren 9 und 10 erläuterten Effekts muß sich der Detektionsort in dem Sinne "zwischen" den Einstrahlungsorten befinden, daß er innerhalb der Grenzgeraden 52,53 liegt, die senkrecht zu der Verbindungslinie 54 beider Einstrahlungsorte 17c,17d durch diese verlaufen. Bevorzugt liegt der Detektionsort in der Nachbarschaft der Verbindungsgeraden 54 und jedenfalls innerhalb eines Grenzkreises 55, der durch beide Einstrahlungsorte verläuft. Auch bei unterschiedlichen Meßabständen D1,D2 beträgt die Phasendifferenz der Modulation des an den beiden Einstrahlungsorten 17a,17b eingestrahlten Primärlichts näherungsweise 180°.

Wie erläutert können die bei der Erfindung zur Ermittlung der Glucosekonzentration bestimmten Meßgrößen mit bekannten meßtechnischen Verfahren gemessen werden. Bei der praktischen Realisierung der Erfindung sind jedoch eine Reihe schwieriger Randbedingungen zu beachten.

Einerseits sind die Genauigkeitsanforderungen sehr hoch. Beispielsweise muß die Phasenverschiebung zwischen dem eingestrahlten Primärlicht und dem detektierten Sekundärlicht mit einer Genauigkeit von typischerweise 0,1° bei einer Frequenz in der Größenordnung von 100 MHz gemessen werden. Dabei muß der Phasenwinkel mit Integrationszeiten von einigen Minuten driftfrei über die gesamte Meßzeit (zumindest über mehrere Stunden) gleichbleiben.

Als Lichtsender werden vorzugsweise Halbleiterlichtsender wie Leuchtdioden oder Laserdioden eingesetzt. Aus der damit erzielbaren geringen Intensität des Primärlichts resultiert eine sehr geringe Empfangsleistung an dem Lichtempfänger in der Größenordnung von nW ($10^{-9}$ Watt).

Das für die erfindungsgemäße Glucoseanalyse verwendete Gerät soll möglichst klein, kompakt und kostengünstig sein. Die meßtechnischen Anforderungen sollen deswegen möglichst ohne teure Spezialbauteile erfüllt werden.

Um die mit diesen Randbedingungen verbundenen Probleme zu lösen, werden im Rahmen der Erfindung vorzugsweise die nachfolgend erörterten Maßnahmen einzeln oder in Kombination miteinander verwendet.

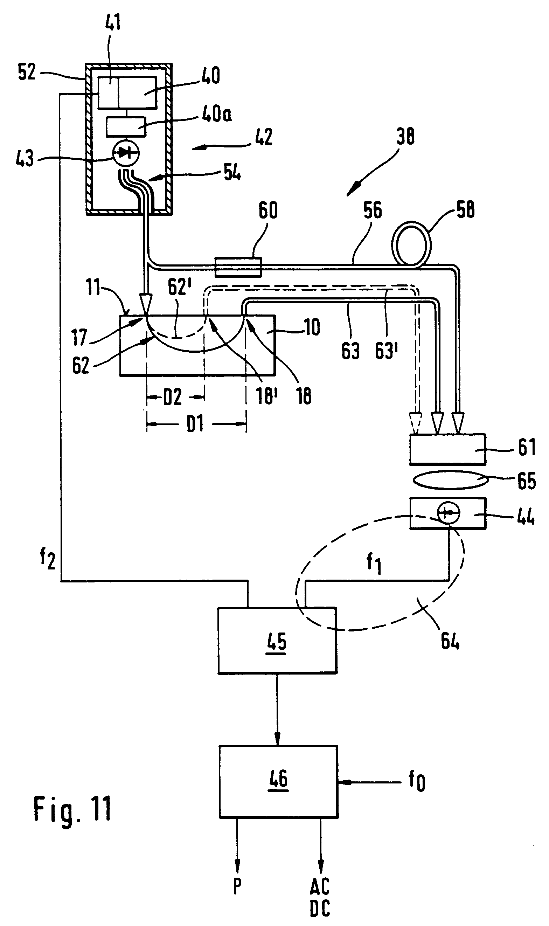

Eine erste Besonderheit der in Figur 11 dargestellten Phasenmeßeinrichtung 38 besteht darin, daß die beiden Frequenzgeneratoren 40, 41, die Treiberstufe 40a und die Primärlichtquelle 43 (bevorzugt eine Laserdiode) in einem elektromagnetisch isolierenden Gehäuse 52 angeordnet sind. Die elektromagnetische Isolierung des Gehäuses 52 erstreckt sich auch auf die Herausführung des Primärlichtes. Der Lichtleiter 15 wird durch einen für die elektromagnetische Hochfrequenzstrahlung praktisch undurchlässigen Kanal (elektronische Falle, electronic pitfall) 54 aus dem Gehäuse 52 herausgeführt. Damit wird erreicht, daß die im Inneren des Gehäuses 52 erzeugte Hochfrequenzleistung (die im Dauerbetrieb in der Größenordnung von 10 mW liegt und im Impulsbetrieb einige Watt erreichen kann) die hochempfindliche Phasenmessung nicht stört. Die Unterbringung der genannten Bauteile in einem elektromagnetisch isolierenden Gehäuse (beispielsweise einem vollständig geschlossenen Metallkasten) ist in diesem Sinne besonders vorteilhaft. Zumindest sollte die Treiberstufe 40a und die Primärlichtquelle 43, vorzugsweise auch der diese ansteuernde Frequenzgenerator 40 elektromagnetisch abgeschirmt sein.

Eine zweite Besonderheit der in Figur 11 dargestellten Phasenmeßeinrichtung 38, die auch bei der Messung anderer Laufzeitparameter vorteilhaft verwendet werden kann, ist darin zu sehen, daß ein Referenzlichtweg 56 vorgesehen ist, durch den das von der Primärlichtquelle 43 erzeugte Licht alternativ zu einem Probenlichtweg 63, der den durch die biologische Matrix 10 führenden Meßlichtweg 62 einschließt, zu dem gleichen Lichtempfänger 44 gelangen kann. Der Referenzlichtweg 56 weist in der dargestellten Ausführungsform eine optische Verzögerungsstrecke 58 (beispielsweise realisiert als Lichtleiter aus Lichtleitfasern) und einen optischen Abschwächer 60 auf. Diese Elemente sind so dimensioniert, daß der Referenzlichtweg 56 an den Probenlichtweg 63 angepaßt ist, wobei die Schwächung und Verzögerung des Lichts auf dem Probenlichtweg im wesentlichen durch den Meßlichtweg 62 innerhalb der biologischen Matrix bestimmt ist. Die Anpassung bedeutet, daß sowohl die Intensität als auch die Laufzeit des von der Primärlichtquelle 43 ausgehenden und auf einem der Lichtwege 56, 63 zu dem Lichtempfänger 44 gelangenden Lichts möglichst ähnlich sein soll. Vorzugsweise wird die Abschwächung und Verzögerung in dem Referenzlichtweg 56 auf die in der Praxis bei dem gegebenen Meßlichtweg 62 unter Meßbedingungen vorkommenden mittleren Werte eingestellt. Hinsichtlich der Lichtintensität sollte die entsprechende Abweichung höchstens +/- 50 % betragen. Die Verzögerung auf dem Referenzlichtweg 56 sollte an die Verzögerung auf dem Probenlichtweg 62 soweit angeglichen sein, daß die Phasenverschiebung auf beiden Wegen sich um maximal +/- 15°, bevorzugt +/- 5°, unterscheidet. Nach vorliegenden experimentellen Ergebnissen erfordert die Anpassung der Lichtwege eine Verzögerungsstrecke 58, die etwa viermal so groß wie der geometrische Abstand (Meßabstand) von Einstrahlungsort 17 und Detektionsort 18 ist. Die Verzögerungsstrecke ist dabei definiert durch die Differenz der Länge des Referenzlichtweges 56 im Vergleich zu dem außerhalb der Probe verlaufenden Teil des Probenlichtweges 63 (d.h. Probenlichtweg 63 abzüglich Meßlichtweg 62).

Der Referenzlichtweg 56 kann mit verhältnismäßig einfachen Mitteln hochstabil aufgebaut werden. Dadurch kann hinsichtlich der durch Änderungen der Glucosekonzentration verursachten Änderungen der Laufzeit mit relativ einfachen und damit kostengünstigen Mitteln eine erhöhte Empfindlichkeit erreicht werden. Wichtig ist dabei, daß der Probenlichtweg 63 und der Referenzlichtweg 56 auf optischem Wege auf den gleichen Lichtempfänger 44 umschaltbar sind. Dies ist in der dargestellten Ausführungsform mit Hilfe eines LCD-optischen Schalters 61 und einer Linse 65 realisiert.

Wenn der Laufzeitparameter (insbesondere die Phasenverschiebung) und gegebenenfalls auch die AC-Amplitude und die DC-Amplitude bei mehreren unterschiedlichen Meßabständen zwischen Einstrahlungsort 17 und Detektionsort 18 bestimmt werden sollen, kann dies grundsätzlich durch Bewegung der den Einstrahlungsort 17 und/oder den Detektionsort 18 bestimmenden Bauteile entlang der Grenzfläche 11 der biologischen Matrix 10 geschehen. Bevorzugt ist jedoch eine Vorrichtung ohne bewegliche Teile. In Figur 11 ist eine Ausführungsform gestrichelt dargestellt, bei der ein im Vergleich zu dem Meßabstand D1 kleinerer Meßabstand D2 mit einem entsprechenden Meßlichtweg 62' dadurch realisiert ist, daß das an dem Detektionsort 18' austretende Licht auf einem weiteren Probenlichtweg 63' (zweckmäßigerweise mit Hilfe einer Lichtleitfaser) dem gleichen LCD-optischen Umschalter 61 zugeführt ist. Dadurch kann die gleiche Phasenmeßvorrichtung 38 verwendet werden, um die Meßgrößen, Phasendifferenz (P), AC-Amplitude und DC-Amplitude für unterschiedliche Meßabstände und (bei Realisierung der hier diskutierten bevorzugten Ausführungsform) einen optischen Referenzlichtweg 56 zu bestimmen.

Eine dritte Besonderheit der in Figur 11 dargestellten Ausführungsform besteht darin, daß der Signalweg 64 zwischen dem Lichtempfänger 44 und dem Signalmischer 45 minimiert ist. Im Rahmen der vorliegenden Erfindung wurde erkannt, daß die Empfindlichkeit der Lichtdetektion durch diese Maßnahme positiv beeinflußt wird. Ein möglichst kurzer Signalweg auf dem Abschnitt zwischen den Bauelementen 44 und 45 führt zu einer geringen Leitungskapazität. Dadurch ist es möglich, trotz der erforderlichen hohen Frequenzen mit einer verhältnismäßig hohen Impedanz des Mischers beziehungsweise eines diesem vorgeschalteten Signalvorverstärkers zu arbeiten, was wiederum hinsichtlich der Signalstärke bei gegebenem Strom der Photodiode vorteilhaft ist. Die nutzbare Empfindlichkeit des an sich einfachen und damit kostengünstigen Halbleiter-Photoempfängers kann somit durch eine einfache Maßnahme erheblich erhöht werden.

Vorzugsweise beträgt der Signalweg zwischen Lichtempfänger 44 und Mischer 45 weniger als 1 cm. Besonders bevorzugt ist der Lichtempfänger 44 und der Signalmischer 45 gemeinsam auf dem gleichen Halbleitersubstrat integriert.

Dabei sind mehrere unterschiedliche schaltungstechnische Realisierungen vorteilhaft. Es kann ein Verstärker mit nachgeschaltetem Mischer (z.B. einem "balanced mixer") eingesetzt werden. Es kann ein FET-Mischer verwendet werden, an dessen Gate der Lichtempfänger 54 direkt angekoppelt ist. Schließlich kann eine als Photoempfänger verwendete Photodiode selbst Bestandteil des Mischers 45 sein. Beispielsweise kann bei einem balanced mixer eine Diode durch die Photodiode ersetzt werden.

Der Lichtempfänger 44 ist besonders bevorzugt eine Avalanche-Photodiode. Eine insbesondere in Verbindung mit einer Avalance-Photodiode bevorzugte Möglichkeit besteht darin, die Empfindlichkeit des Lichtempfängers 44 unmittelbar mit der Frequenz $f_2$ zu modulieren. Dies kann im Falle eines Photomulitpliers dadurch geschehen, daß $f_2$ an dessen Dynode angelegt wird. Im Falle eines Halbleiter-Lichtempfängers kann dessen Versorgungsspannung mit $f_2$ moduliert werden. Dadurch werden die Funktionen des Lichtempfängers 44 und des Mischers 45 in einem Bauteil kombiniert.

Die Genauigkeit der Phasenmessung mit einer Phasenmeßeinrichtung, die nach dem Heterodyn-Prinzip arbeitet, ist wesentlich davon abhängig, daß die Differenzfrequenz $f_0$ zwischen den beiden Frequenzgeneratoren ($f_1$, $f_2$) 40 und 41 ("Kreuzkorrelationsfrequenz") über die Meßzeit konstant bleibt. Da andererseits $f_1$ (beispielsweise 1 KHz) nur einen kleinen Bruchteil der Absolutfrequenzen der Frequenzgeneratoren 40, 41 (beispielsweise 100,00 MHz und 100,001 MHz) ausmacht, müßten in den Frequenzgeneratoren 40, 41 hochstabile und deswegen aufwendige Oszillatoren eingesetzt werden.

Gemäß einer bevorzugten Ausführungsform der Erfindung wird statt dessen die Differenzfrequenz $f_0$ von einem Differenzfrequenzgenerator 66 vorgegeben und die Frequenz des zweiten Meßfrequenzgenerators $f_2$ auf Basis der vorgegebenen Differenzfrequenz so geregelt, daß der zweite Frequenzgenerator 41 Frequenzschwankungen des ersten Frequenzgenerators 40 mit konstanter Differenzfrequenz folgt.

Bei der Ausführungsform gemäß Figur 12 ist dies dadurch realisiert, daß die Frequenzen $f_1$ und $f_2$ der Generatoren 40, 41 an den Eingängen eines Signalmischers 67 anliegen, an dessen Ausgang die jeweils aktuelle Differenzfrequenz $f_{0'}$ erzeugt wird. Diese wird als Ist-Signal einer Regeleinheit 68 zugeführt, welcher andererseits das Signal des Differenzfrequenzgenerators 66 als Sollwert zugeführt wird. Die Regeleinheit 68 vergleicht das Ist-Signal $f_{0'}$ mit dem Sollsignal $f_0$ und erzeugt ein Korrektursignal k, welches einem der beiden Frequenzgeneratoren, in der Figur dem Frequenzgenerator 41 zugeführt wird, um dessen Frequenz $f_2$ so zu regeln, daß die Differenz zwischen $f_1$ und $f_2$ exakt der vorgegebenen Differenzfrequenz $f_0$ entspricht. Die Regelung erfolgt bevorzugt in bekannter Weise mit dem PLL-Prinzip.

Die anhand der Figuren 11 und 12 erläuterten meßtechnischen Besonderheiten sind auch für andere Aufgaben, bei denen Meßgrößen wie die Phasendifferenz P, die AC-Amplitude und die DC-Amplitude an einem streuenden Medium gemessen werden sollen, um dessen Lichttransporteigenschaften zu bestimmen, vorteilhaft verwendbar.

**Patentansprüche**

1. Verfahren zur analytischen Bestimmung der Konzentration von Glucose in einer biologischen Matrix, bei welchem

   in einem Detektionsschritt Licht von einem Lichtsender durch eine die biologische Matrix begrenzende Grenzfläche als Primärlicht in diese eingestrahlt und aus der biologischen Matrix durch eine diese begrenzende Grenzfläche austretendes Licht von einem Lichtempfänger detektiert wird, um eine durch die Wechselwirkung mit der biologischen Matrix veränderliche meßbare physikalische Eigenschaft des Lichtes zu bestimmen, die mit der Konzentration von Glucose in der biologischen Matrix korreliert und

   in einem Auswerteschritt die Glucosekonzentration auf Basis der Änderung der bei mindestens einem Detektionsschritt bestimmten physikalischen Eigenschaft des Lichts im Vergleich zu einer Kalibration ermittelt wird, **dadurch gekennzeichnet**, daß

   in dem Detektionsschritt als mit der Glucose-Konzentration korrelierende meßbare Eigenschaft ein der Laufzeit des Lichtes innerhalb der biologischen Matrix zwischen einem definierten Einstrahlungsort und einem definierten Detektionsort entsprechender Parameter bestimmt wird, der mit der Laufzeit korreliert und unter den jeweiligen Meßbedingungen überwiegend von der Laufzeit des Lichts zwischen dem Einstrahlungsort und dem Detektionsort abhängt.

2. Verfahren nach Anspruch 1, **dadurch gekennzeichnet**, daß der Meßabstand zwischen dem Einstrahlungsort und dem Detektionsort kleiner als 4 cm, bevorzugt kleiner als 3 cm und besondere bevorzugt kleiner als 2 cm ist.

3. Verfahren nach einem der vorhergehenden Ansprüche, **dadurch gekennzeichnet**, daß der der Laufzeit des Lichtes entsprechende Parameter bei mehreren unterschiedlichen Meßabständen zwischen Einstrahlungsort und Detektionsort bestimmt und in dem Auswerteschritt die Abhängigkeit der Laufzeit des Lichtes von dem Meßabstand zur Ermittlung der Glucosekonzentration verwendet wird.

4. Verfahren nach einem der vorhergehenden Ansprüche, **dadurch gekennzeichnet**, daß in dem Detektionsschritt zur direkten Bestimmung der Laufzeit ein kurzer Impuls des Primärlichtes in die biologische Matrix eingestrahlt und der resultierende an dem Detektionsort austretende Impuls detektiert wird.

5. Verfahren nach einem der Ansprüche 1 bis 3, **dadurch gekennzeichnet**, daß in dem Detektionsschritt das Primärlicht mit einer hochfrequenten Trägerfrequenz moduliert und die Phasenverschiebung des Sekundärlichts gegenüber dem Primärlicht als der Laufzeit entsprechender Parameter bestimmt wird.

6. Verfahren nach Anspruch 5, **dadurch gekennzeichnet**, daß in dem Detektionsschritt die Änderung der AC-Amplitude der Modulation des Sekundärlichtes in Relation zu der AC-Amplitude der Modulation des Primärlichtes bestimmt und zusätzlich zu der Phasenverschiebung in dem Auswerteschritt zur Ermittlung der Glucosekonzentration verwendet wird.

7. Verfahren nach einem der Ansprüche 5 oder 6, **dadurch gekennzeichnet**, daß in dem Detektionsschritt die Änderung der DC-Amplitude des Sekundärlichtes in Relation zu der DC-Amplitude des Primärlichtes bestimmt und zusätzlich zu der Phasenverschiebung in dem Auswerteschritt zur Ermittlung der Glucosekonzentration verwendet wird.

8. Verfahren nach einem der Ansprüche 5 bis 7, **dadurch gekennzeichnet**, daß in dem mindestens einen Detektionsschritt das Primärlicht mit mehreren unterschiedlichen Trägerfrequenzen moduliert und jeweils die Phasenverschiebung des Sekundärlichtes gegenüber dem Primärlicht zur Bestimmung der mittleren optischen Weglänge gemessen wird.

9. Verfahren nach einem der Ansprüche 5 bis 8, **dadurch gekennzeichnet**, daß in dem mindestens einen Detektionsschritt das Primärlicht gleichzeitig an zwei verschiedenen Einstrahlungsorten so eingestrahlt wird, daß sich die von den beiden Einstrahlungsorten ausgehenden, in der biologischen Matrix propagierenden Lichtintensitätswellen überlagern und eine von der Laufzeit des Lichts zwischen den Einstrahlungsorten und einem definierten Detektionsort abhängige aus der Überlagerung resultierende Änderung des an dem definierten Detektionsort detektierten Lichtes als der Laufzeit entsprechender Parameter in dem Detektionsschritt bestimmt und in dem Auswerteschritt zur Ermittlung der Glucosekonzentration verwendet wird.

10. Verfahren nach einem der vorhergehenden Ansprüche, **dadurch gekennzeichnet**, daß die Temperatur an dem Detektionsort vorzugsweise berührungslos gemessen und in dem Auswertealgorithmus berücksichtigt wird.

11. Verfahren nach einem der vorhergehenden Ansprüche, **dadurch gekennzeichnet**, daß die Temperatur an dem Detektionsort konstant gehalten wird.

12. Vorrichtung zur Bestimmung der Konzentration von Glucose in einer biologischen Matrix, insbesondere zur Durchführung des Verfahrens nach einem der vorhergehenden Ansprüche, mit

einem zum Anlegen an eine Grenzfläche der biologischen Matrix vorgesehenen Meßkopf (12), Einstrahlungsmitteln (42) mit einem Primärlichtsender (43) zum Einstrahlen von Primärlicht in die biologische Matrix (10) durch eine diese begrenzende Grenzfläche (11), Detektionsmitteln (48) zum Detektieren von aus der biologischen Matrix (10) durch eine diese begrenzende Grenzfläche (11) austretendem Sekundärlicht und zur Bestimmung einer durch die Wechselwirkung des Lichts mit der biologischen Matrix (10) veränderlichen physikalischen Eigenschaft des Lichts, die mit der Konzentration von Glucose in der biologischen Matrix (10) korreliert und

Auswertemitteln (50) zur Ermittlung der Glucosekonzentration aus der bestimmten physikalischen Eigenschaft des Lichtes, **dadurch gekennzeichnet**, daß die Detektionsmittel (48) Mittel (14,38) zur Messung eines der Laufzeit des Lichtes auf einem Meßlichtweg (62) innerhalb der biologischen Matrix (10) zwischen einem definierten Einstrahlungsort (17) und einem definierten Detektionsort (18) entsprechenden Parameters als mit der Glucosekonzentration korrelierende physikalische Eigenschaft des Lichtes einschließen, der mit der Laufzeit korreliert und unter den jeweiligen Meßbedingungen überwiegend von der Laufzeit des Lichts zwischen dem Einstrahlungsort und dem Detektionsort abhängt.

13. Vorrichtung nach Anspruch 12, **dadurch gekennzeichnet**, daß

die Einstrahlungsmittel (42) einen Meßfrequenzgenerator (40) und eine Treiberstufe (40a) zur Steuerung der Primärlichtquelle (43) einschließen, wodurch das Primärlicht mit einer hochfrequenten Trägerfrequenz moduliert ist und die Mittel zur Messung eines der Laufzeit des Lichtes in der biologischen Matrix entsprechenden Parameters eine Phasenmeßeinrichtung (45,46,47) einschließen, um die Phasenverschiebung des Sekundärlichtes gegenüber dem Primärlicht als mit der Laufzeit des Lichtes korrelierenden Parameter zu bestimmen.

14. Vorrichtung nach Anspruch 13, **dadurch gekennzeichnet**, daß die Treiberstufe (40a) und die Primärlichtquelle (43) in einem elektromagnetisch isolierenden Gehäuse angeordnet sind.

15. Vorrichtung nach Anspruch 14, **dadurch gekennzeichnet**, daß auch der Meßfrequenzgenerator (40) in einem elektromagnetisch isolierenden Gehäuse (52) angeordnet ist.

16. Vorrichtung nach einem der Ansprüche 13 bis 15, **dadurch gekennzeichnet**, daß die Phasenmeßeinrichtung (38) einen Signalmischer (45) aufweist, dem das Ausgangssignal des Lichtempfängers (44) zugeführt wird und der Signalweg (64) zwischen dem Lichtempfänger (44) und dem Signalmischer (45) minimiert ist.

17. Vorrichtung nach Anspruch 16, **dadurch gekennzeichnet**, daß der Lichtempfänger (44) und der Signalmischer (45) gemeinsam auf dem gleichen Halbleitersubstrat integriert sind.

18. Vorrichtung nach Anspruch 17, **dadurch gekennzeichnet**, daß der Lichtempfänger (44) eine Photodiode ist und die Photodiode ein Bestandteil des Signalmischers (45) ist.

19. Vorrichtung nach einem der Ansprüche 12 bis 18, **dadurch gekennzeichnet**, daß die Mittel zur Messung eines der Laufzeit des Lichts auf dem Meßlichtweg (62) innerhalb der biologischen Matrix entsprechenden Parameters eine optische Referenzlichtstrecke (56) einschließen, die hinsichtlich der Lichtlaufzeit und der Lichtintensitätsschwächung an den Meßlichtweg (62) angepaßt ist, wobei das Meßlicht zwischen einem den Meßlichtweg (62) einschließenden Probenlichtweg (63) und dem Referenzlichtweg (56) optisch umschaltbar ist.

20. Vorrichtung nach einem der Ansprüche 13 bis 19, **dadurch gekennzeichnet**, daß die Phasenmeßeinrichtung (38) einen ersten und einen zweiten mit konstanter Differenz oszillierenden Meßfrequenzgenerator (40,41) aufweist, wobei die Differenzfrequenz zwischen den Frequenzen des ersten Frequenzgenerators (40) und des zweiten Frequenzgenerators (41) von einem Differenzfrequenzgenerator (66) vorgegeben und die Frequenz des zweiten Meßfrequenzgenerators (41) auf Basis der vorgegebenen Differenzfrequenz so geregelt wird, daß der zweite Frequenzgenerator (41) Frequenzschwankungen des ersten Frequenzgenerators (40) mit konstanter Differenzfrequenz folgt.

**Claims**

1. A method for analytically determining the glucose concentration in a biological matrix, comprising

    a detection step in which light from a light emitter is irradiated as primary light through a boundary surface into the biological matrix and light emerging from said matrix through said boundary surface is detected by a light sensor in order to determine a measurable physical property of the light that is changed by interaction with the biological matrix, said property correlating with the glucose concentration in the biological matrix, and
    in an evaluation step the glucose concentration is determined on the basis of the change of the physical property of the light, as detected in at least one detection step, in comparison with a calibration,
        characterized in that
    in the detection step a parameter corresponding to the light transit-time inside the biological matrix between a defined irradiation site and a defined detection site is measured as the measurable property correlating with the glucose concentration, which parameter correlates with the transit-time and depends under the particular conditions predominantly on the light transit-time between the irradiation site and the detection site.

2. A method according to claim 1, characterized in that the measurement distance between the irradiation site and the detection site is less than 4 cm, preferably less than 3 cm and especially-preferred less than 2 cm.

3. A method according to one of the preceding claims, characterized in that the parameter corresponding to the light transit-time is determined at several different measurement distances between the irradiation site and the detection site and in that in the evaluation step the dependence of the light transit-time on the measurement distance is used to determine the glucose concentration.

4. A method according to one of the preceding claims, characterized in that in the detection step a short pulse of primary light is irradiated into the biological matrix and the resulting pulse emerging at the detection site is detected in order to directly determine the transit-time.

5. A method according to one of claims 1 through 3, characterized in that the primary light is modulated with a high-frequency carrier frequency and in that the phase-shift of the secondary light relative to the primary light is determined as the parameter corresponding to the transit-time.

6. A method according to claim 5, characterized in that in the detection step the change in AC amplitude of the modulation of the secondary light is determined in relation to the AC amplitude of the modulation of the primary light and said change of AC amplitude is used additionally to the phase shift in the evaluation step to determine the glucose concentration.

7. A method according to either of claims 5 and 6, characterized in that in the detection step the change of the DC amplitude of the secondary light is determined in relation to the DC amplitude of the primary light and said change of DC amplitude is used in addition to the phase shift in the evaluation step to determine the glucose concentration.

8. A method according to one of claims 5 through 7, characterized in that in the at least one detection step the primary light is modulated with a plurality of different carrier frequencies and in each case the respective phase shift of the secondary light is measured relative to the primary light as a measure of the mean optical path-length.

9. A method according to one of claims 5 through 8, characterized in that in at least one detection step the primary light is simultaneously irradiated at two different irradiation sites in such manner that the light intensity waves propagating from the two irradiation sites through the biological matrix are superposed and a change of the light detected at a defined detection site resulting from said superposition and depending on the light transit-time between the irradiation sites and the defined detection site is determined as the parameter corresponding to the transit-time in the detection step and is used in the evaluation step to determine the glucose concentration.

10. A method according to one of the preceding claims, characterized in that the temperature of the detection site is measured preferably without contact and is taken into account in the evaluation algorithm.

11. A method according to one of the preceding claims, characterized in that the detection-site temperature is kept constant.

12. Apparatus for determining the glucose concentration in a biological matrix, in particular to implement the method according to one of the preceding claims, comprising a measuring head (12) to be placed against a boundary surface of the biological matrix, irradiation means (42) with a primary light emitter (43) for irradiating primary light through a boundary surface (11) into the biological matrix (10), detection means (48) for detecting secondary light emerging from said biological matrix (10) through said boundary surface (11) and for determining a physical property of the light that is changed by interaction with the biological matrix (10) said property correlating with the glucose concentration in the biological matrix (10), and evaluation means (50) for determining the glucose concentration from the determined physical property of the light,
characterized in that
the detection means (48) comprise means (14, 38) for measuring a parameter corresponding to the light transit-time along a measurement light path (62) within the biological matrix (10) between a defined irradiation site (17) and a defined detection site (18) as a physical property of the light which correlates with the glucose concentration, which parameter correlates with the transit-time and depends under the particular conditions predominantly on the light transit-time between the irradiation site and the detection site.

13. Apparatus according to claim 12, characterized in that the irradiation means (42) comprise a measurement frequency generator (40) and a driver stage (40a) controlling the primary light emitter (43) thereby to modulate the primary light with a high-frequency carrier frequency, and in that the means for measuring a parameter corresonding to the light transit-time in the biological matrix comprise a phase measurement device (45, 46, 47) for determining the phase shift of the secondary light relative to the primary light as a parameter correlating with the light transit-time.

14. Apparatus according to claim 13, characterized in that the driver stage (40a) and the primary light emitter (43) are enclosed in an electromagnetically insulating housing.

15. Apparatus according to claim 14, characterized in that the measurement frequency generator (40) also is enclosed in an electromagnetically insulating housing (52).

16. Apparatus according to one of claims 13 through 15, characterized in that the phase measurement device (38) comprises a signal mixer (45) receiving the output signal from the light sensor (44) and in that the signal path (64) between the light sensor (44) and the signal mixer (45) is minimized.

17. Apparatus according to claim 16, characterized in that the light sensor (44) and the signal mixer (45) are jointly integrated on the same semiconductor substrate.

18. Apparatus according to claim 17, characterized in that the light sensor (44) is a photodiode and in that the photodiode is a part of the signal mixer (45).

19. Apparatus according to one of claims 12 through 18, characterized in that the means for measuring a parameter corresponding to the light transit-time along the measurement light path (62) within the biological matrix comprise an optical reference light path (56) which is adapted to the measurement light path (62) regarding light transit-time and light intensity attenuation, wherein the measurement light is optically switchable between a sample light path (63) comprising the measurement light path (62) and the reference light path (56).

20. Apparatus according to one of claims 13 through 19, characterized in that the phase measurement device (38) comprises a first and a second measuring frequency generator (40, 41) oscillating at a constant differential frequency wherein the differential frequency between the frequencies of the first frequency generator (40) and the second frequency generator (41) is predetermined by means of a difference-frequency generator (66) and the frequency of the second measurement frequency generator (41) is regulated on the basis of the predetermined differential frequency in such manner that the second frequency generator (41) follows the frequency fluctuations of the first frequency generator (40) at a constant differential frequency.

**Revendications**

1. Procédé de détermination analytique de la concentration de glucose dans une matrice biologique, comprenant

   une étape de détection dans laquelle on soumet la matrice biologique à une irradiation avec une lumière issue d'une source lumineuse, en tant que lumière primaire, à travers une surface délimitant cette matrice biologique et on détecte, au moyen d'un capteur de lumière, la lumière émergeant de la matrice biologique à travers la surface qui délimite celle-ci, afin de déterminer une caractéristique physique mesurable de la lumière qui varie par interaction avec la matrice biologique et qui est en corrélation avec la concentration de glucose dans la matrice biologique, et

   dans une étape d'évaluation, on détermine la concentration de glucose en se basant sur la variation de la caractéristique physique de la lumière détectée dans au moins une étape de détection, par comparaison avec un étalonnage,
   caractérisé en ce que

   dans l'étape de détection, en tant que caractéristique mesurable en corrélation avec la concentration de glucose, on mesure un paramètre correspondant au temps de passage de la lumière à travers la matrice biologique entre un site d'irradiation défini et un site de détection défini, ce paramètre étant en corrélation avec le temps de passage et dépendant, dans les conditions de mesure données, essentiellement du temps de passage de la lumière entre le site d'irradiation et le site de détection.

2. Procédé selon la revendication 1, caractérisé en ce que la distance de mesure entre le site d'irradiation et le site de détection est inférieure à 4 cm, de préférence inférieure à 3 cm et en particulier, inférieure à 2 cm.

3. Procédé selon l'une quelconque des revendications précédentes, caractérisé en ce que l'on détermine le paramètre correspondant au temps de passage de la lumière pour plusieurs distances de mesure différentes entre le site d'irradiation et le site de détection et que dans l'étape d'évaluation, on utilise la variation du temps de passage de la lumière en fonction de la distance de mesure pour déterminer la concentration de glucose.

4. Procédé selon l'une quelconque des revendications précédentes, caractérisé en ce que dans l'étape de détection, pour la détermination directe du temps de passage, on irradie la matrice biologique avec une brève impulsion de la lumière primaire et on détecte l'impulsion résultante, sortant au niveau du site de détection.

5. Procédé selon l'une quelconque des revendications 1 à 3, caractérisé en ce que dans l'étape de détection, on module la lumière primaire avec une fréquence porteuse située dans la gamme des hautes fréquences et que l'on détermine le déphasage entre la lumière secondaire et la lumière primaire en tant que paramètre correspondant au temps de passage.

6. Procédé selon la revendication 5, caractérisé en ce que dans l'étape de détection, on détermine la variation de l'amplitude CA de la modulation de la lumière secondaire en relation avec l'amplitude CA de la modulation de la lumière primaire et dans l'étape d'évaluation, on utilise cette variation, en plus du déphasage, pour la détermination de la concentration de glucose.

7. Procédé selon l'une quelconque des revendications 5 ou 6, caractérisé en ce que dans l'étape de détection, on détermine la variation de l'amplitude CC de la modulation de la lumière secondaire en relation avec l'amplitude CC de la modulation de la lumière primaire et dans l'étape d'évaluation, on utilise cette variation en plus du déphasage pour la détermination de la concentration de glucose.

8. Procédé selon l'une quelconque des revendications 5 à 7, caractérisé en ce que dans au moins une étape de détection, on module la lumière primaire avec plusieurs fréquences porteuses différentes et que l'on mesure chaque fois le déphasage entre la lumière secondaire et la lumière primaire pour déterminer la longueur du trajet optique moyenne de la lumière.

9. Procédé selon l'une quelconque des revendications 5 à 8, caractérisé en ce que dans au moins une étape de détection, on irradie simultanément deux différents sites d'irradiation avec la lumière primaire de telle façon que les ondes d'intensité lumineuse partant des deux sites d'irradiation, se propageant à travers la matrice biologique, se superposent et dans l'étape de détection, on détermine la variation, résultant de la superposition, dépendant du temps de passage entre les sites d'irradiation et le site de détection défini, de la lumière détectée au site de détection défini, en tant que paramètre correspondant au temps de passage et dans l'étape d'évaluation, on utilise cette variation pour la détermination de la concentration de glucose.

10. Procédé selon l'une quelconque des revendications précédentes, caractérisé en ce que l'on mesure la température au site de détection de préférence sans contact et on la prend en compte dans l'algorithme d'évaluation.

11. Procédé selon l'une quelconque des revendications précédentes, caractérisé en ce qu'au site de détection, on maintient une température constante.

12. Dispositif destiné à la détermination de la concentration de glucose dans une matrice biologique, en particulier à la mise en oeuvre du procédé selon t'une quelconque des revendications précédentes, comprenant

une tête de mesure (12) prévue pour être placée sur une surface délimitant la matrice biologique,

des moyens d'irradiation (42) comprenant un émetteur de lumière primaire (43) destiné à irradier la lumière primaire à travers la matrice biologique (10), à travers une surface (11) délimitant la matrice biologique,

des moyens de détection (48) destinés à détecter la lumière secondaire émergeant de la matrice biologique (10) à travers la surface (11) délimitant celle-ci et à déterminer une caractéristique physique de la lumière, variant en fonction de l'interaction de la lumière et de la matrice biologique (10), qui est en corrélation avec la concentration de glucose dans la matrice biologique (10), et

des moyens d'évaluation (50) destinés à évaluer la concentration de glucose à partir de la caractéristique physique de la lumière que l'on a déterminée,
caractérisé en ce que

les moyens de détection (48) comprennent des moyens (14,38) destinés à mesurer un paramètre correspondant au temps de passage de la lumière suivant un trajet (62) de la lumière mesurée à l'intérieur de la matrice biologique (10) entre un site d'irradiation (17) défini et un site de détection (18) défini, en tant que caractéristique physique de la lumière en corrélation avec la concentration de glucose, ce paramètre étant en corrélation avec le temps de passage et dépendant, dans les conditions de mesure données, essentiellement du temps de passage de la lumière entre le site d'irradiation et le site de détection.

13. Dispositif selon la revendication 12, caractérisé en ce que

les moyens d'irradiation (42) comprennent un générateur (40) de fréquence de mesure et un excitateur (40a) destinés à commander la source de lumière primaire (43), la lumière primaire étant ainsi modulée avec une fréquence porteuse située dans la gamme des hautes fréquences et que
les moyens destinés à mesurer un paramètre correspondant au temps de passage de la lumière à travers la matrice biologique comprennent un appareil de mesure de phase (45, 46, 47) destiné à déterminer le déphasage entre la lumière secondaire et la lumière primaire en tant que paramètre en corrélation avec le temps de passage de la lumière.

14. Dispositif selon la revendication 13, caractérisé en ce que l'excitateur (40a) et la source de lumière primaire (43) sont disposés dans un boitier assurant une isolation vis-à-vis des ondes électromagnétiques.

15. Dispositif selon la revendication 14, caractérisé en ce que le générateur (40) de fréquence de mesure est également situé dans un boitier (52) assurant une isolation vis-à-vis des ondes électromagnétiques.

16. Dispositif selon l'une quelconque des revendications 13 à 15, caractérisé en ce que l'appareil de mesure de phase (38) présente un mélangeur de signal (45) recevant le signal de sortie du capteur de lumière (44) et le trajet (64) du signal entre le capteur de lumière (44) et le mélangeur de signal (45) est réduit au minimum.

17. Dispositif selon la revendication 16, caractérisé en ce que le capteur de lumière (44) et le mélangeur de signal (45) sont intégrés conjointement dans le même substrat semiconducteur.

18. Dispositif selon la revendication 17, caractérisé en ce que le capteur de lumière (44) est une photodiode et que la photodiode est un constituant du mélangeur de signal (45).

19. Dispositif selon l'une quelconque des revendications 12 à 18, caractérisé en ce que les moyens destinés à mesurer un paramètre correspondant au temps de passage de la lumière suivant le trajet (62) de mesure de la lumière à travers la matrice biologique comprennent un trajet (56) de lumière de référence optique qui est ajusté au trajet (62) de mesure de la lumière du point de vue du temps de passage de la lumière et de l'affaiblissement de l'intensité lumineuse, la lumière mesurée pouvant subir une commutation optique entre le trajet (63) de la lumière à tester, qui comprend le trajet (62) de mesure de la lumière, et le trajet (56) de lumière de référence.

20. Dispositif selon l'une quelconque des revendications 13 à 19, caractérisé en ce que les moyens de mesure de phase (38) présentent un premier et un second générateur de fréquence de mesure (40, 41), oscillant avec une différence constante, la fréquence différencielle entre la fréquence du premier générateur de fréquence (40) et celle du second générateur de fréquence (41) étant prédéterminée par un générateur de fréquence différentielle (66) et la fréquence du second générateur de fréquence de mesure (41) étant réglée, en se basant sur la fréquence différentielle prédéterminée, de façon que le second générateur de fréquence (41) suive les oscillations de fréquence du premier générateur de fréquence (40) avec une fréquence différentielle constante.

Fig.1

EP 0 726 729 B1

Fig. 2

Fig. 3

Fig. 4

# Fig. 5

# Fig. 7

Fig. 6

Fig. 8

Fig.9

Fig. 10

Fig. 11

Fig. 12